# EUROPEAN PATENT APPLICATION

(11) **EP 4 708 305 A1**
(43) Date of publication of application: **11.03.2026**
(21) Application number: 25199199.8
(22) Date of filing: 29.08.2025
(51) Int. Cl.: G16C 20/40, G16C 20/90, G16C 20/20, G06F 16/901, G06F 16/907

(54) **HIERARCHY-BASED CHEMICAL COMPOUND LIBRARY IDENTIFIER GENERATION**

(30) Priority: 30.08.2024 US 202418820803
(71) Applicant: HighChem s.r.o., 821 09 Bratislava (SK)
(72) Inventor: BENKOVIC, Samuel, Bratislava (SK); UHLÁR, Marek, Partizánske (SK); HALENÁR, Milan, Nitra (SK)
(74) Representative: Boult Wade Tennant LLP

(57) **Abstract**

Embodiments described herein relate to a process for generation of annotation-accessible library spectral content. A system can comprise a memory that stores, and a processor that executes, computer executable components. The computer executable components can comprise an identifying component that identifies chemical compound data describing a chemical compound, and a generating component that generates an identifier from the chemical compound data based on an annotation type of the compound data as compared to an annotation ranking schema.

## Description

### BACKGROUND

A network or library of spectral data can be employed for various purposes, including determining similarities, differences and/or relationships between different spectral content for different compounds, whether molecules or more complex compounds. Search queries directed to such spectral data often exploit metadata, such as identifiers comprising annotations describing, referencing and/or corresponding to different library spectral content.

### BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 illustrates a block diagram of an example scientific instrument for performing one or more operations, in accordance with one or more embodiments described herein.
FIG. 2 illustrates a flow diagram of an example method of performing operations using the scientific instrument of FIG. 1, in accordance with one or more embodiments described herein.
FIG. 3 illustrates a graphical user interface (GUI) that can be used in the performance of one or more of the methods described herein, in accordance with one or more embodiments described herein.
FIG. 4 illustrates a block diagram of an example computing device that can perform one or more of the methods disclosed herein, in accordance with one or more embodiments described herein.
FIG. 5 illustrates a block diagram of an example, non-limiting system that can facilitate a process for library spectral content identifier generation, in accordance with one or more embodiments described herein.
FIG. 6 illustrates a block diagram of another example, non-limiting system that can facilitate a process for library spectral content identifier generation, in accordance with one or more embodiments described herein.
FIG. 7 illustrates a flow diagram of an identifier generation workflow that can be performed by the non-limiting system of FIG. 6, in accordance with one or more embodiments described herein.
FIG. 8 illustrates a flow diagram of an identifier generation data flow that can be performed by the non-limiting system of FIG. 6, in accordance with one or more embodiments described herein.
FIG. 9 illustrates a diagram of an exemplary annotation ranking schema that can be employed by the non-limiting system of FIG. 6, in accordance with one or more embodiments described herein.
FIG. 10 illustrates a flow diagram of one or more processes that can be performed by the identifier generation system of FIG. 5, in accordance with one or more embodiments described herein.
FIG. 11 illustrates another flow diagram of one or more processes that can be performed by the identifier generation system of FIG. 6, in accordance with one or more embodiments described herein.
FIG. 12 illustrates a continuation of the flow diagram of FIG. 11 of one or more processes that can be performed by the identifier generation system of FIG. 6, in accordance with one or more embodiments described herein.
FIG. 13 illustrates a block diagram of example scientific instrument system in which one or more of the methods described herein can be performed, in accordance with one or more embodiments described herein.
FIG. 14 illustrates a block diagram of an example operating environment into which embodiments of the subject matter described herein can be incorporated.
FIG. 15 illustrates an example schematic block diagram of a computing environment with which the subject matter described herein can interact and/or be implemented at least in part.

### SUMMARY

The following presents a summary to provide a basic understanding of one or more embodiments described herein. This summary is not intended to identify key or critical elements, and/or to delineate scope of particular embodiments or scope of claims. Its sole purpose is to present concepts in a simplified form as a prelude to the more detailed description that is presented later. In one or more embodiments, systems, computer-implemented methods, apparatuses and/or computer program products described herein can provide a plug-and-play process for generating identifiers, and/or updating a library datastore with such identifiers, based at least partially on an annotation ranking schema.

In accordance with an embodiment, a system can comprise a memory that stores computer executable components, and a processor that executes the computer executable components. The computer executable components can comprise an identifying component that identifies chemical compound data describing a chemical compound, and a generating component that generates an identifier from the chemical compound data based on an annotation type of the compound data as compared to an annotation ranking schema.

In accordance with another embodiment, a computer-implemented method can comprise identifying, by a system operatively coupled to a processor, chemical compound data describing a chemical compound, and generating, by the system, an identifier from the chemical compound data based on an annotation type of the compound data as compared to an annotation ranking schema.

In accordance with still another embodiment, a computer program product facilitates a process for generating chemical compound identifiers based on varying annotation types, the program instructions executable by a processor to cause the processor to identify, by the processor, chemical compound data describing a chemical compound, and generate, by the processor, an identifier from the chemical compound data based on an annotation type of the compound data as compared to an annotation ranking schema.

The one or more embodiments described herein can be employed with varying annotation types comprising varying text, code and/or metadata. Indeed, a benefit of the one or more embodiments described herein is an ability to differentiate between such varying annotation types for generating the one or more identifiers.

In one or more embodiments, the annotation ranking schema employed to prioritize the different annotation types for a same compound or spectrum for a compound, can be customized at any suitable time to adjust ranking data employed to provide rankings for the varying annotation types of input compound data (e.g., input to a non-limiting system described herein).

The one or more embodiments described herein can be implemented within, in connection with and/or coupled to a scientific imaging device.

The one or more embodiments described herein can be applied on a plug-and-play basis to various architectures of existing spectral library and/or library datastores of spectral data. That is, the one or more embodiments described herein can generate identifiers for compounds (including for spectra corresponding to compounds) regardless of data structure of a spectral library and/or library datastore.

The one or more embodiments described herein can provide for consistency of content of identifiers generated for same compounds but based on different annotation types. That is, the one or more identifiers generated for a same compound can be consistent such as to not be in conflict with one another (e.g., describing the compound as having conflicting properties, for example). This can be facilitated by the use of the annotation ranking schema and various cross-checks and/or comparisons performed by the one or more embodiments described herein.

The one or more embodiments described herein can be employed to generate the identifiers such that a library updated with the identifiers can be searched in an annotation type-agnostic manner. That is, information can be returned in response to a query that is based on different annotation types (e.g., plural annotation types) for a same compound. Accordingly, a search employing one annotation type can return data from another annotation type that has already been cross-checked for consistency and/or labeled for ranking based on the annotation ranking schema.
Further aspects of the invention are set out in the following numbered paragraphs:
Clause 1. A system, comprising:
   a memory that stores computer executable components; and
   a processor that executes the computer executable components stored in the memory, wherein the computer executable components comprise:
      an identifying component that identifies chemical compound data describing a chemical compound; and
      a generating component that generates an identifier from the chemical compound data based on an annotation type of the compound data as compared to an annotation ranking schema.
Clause 2. The system of clause 1,
   wherein the identifying component identifies plural aspects of the chemical compound data having varying annotation types, including the annotation type, and
   wherein the computer executable components further comprise a selecting component that selects a first aspect, of the plural aspects, and having the annotation type, to employ for the identifier based on the annotation type being a highest ranked annotation type, of the varying annotation types, based on the annotation ranking schema.
Clause 3. The system of clause 2, wherein the computer executable components further comprise:
   a prioritizing component that prioritizes the plural aspects according to the varying annotation types as compared to the annotation ranking schema.
Clause 4. The system of clause 2,
   wherein the generating component further generates a second identifier based on a lesser ranking second annotation type, of the varying annotation types, as compared to the annotation ranking schema, and
   wherein a second content of the second identifier and a content of the identifier consistently describe a same property of the chemical compound.
Clause 5. The system of clause 1, wherein the computer executable components further comprise:
   an updating component that updates a library datastore comprising library identifiers for chemical compounds, including the chemical compound, with the identifier for the chemical compound; and
   an executing component that directs a query corresponding to the chemical compound, to the library datastore, and returns, for the same query, plural identifiers, including the identifier, having been generated for the same chemical compound based on the annotation ranking schema.
Clause 6. The system of clause 1, wherein the computer executable components further comprise:
   a comparing component that compares the identifier and a library identifier for the chemical compound, of a library datastore; and
   an updating component that determines whether to update the library datastore with the identifier based on a first determination resolving a first comparison of the annotation type corresponding to the identifier and a second annotation type corresponding to the library identifier.
Clause 7. The system of clause 6,
   wherein the first comparison comprises a first sub-comparison of forms of the annotation type and the second annotation type, a second sub-comparison of rankings of the annotation type and the second annotation type as compared to the annotation ranking schema, or both.
Clause 8. The system of clause 6,
   wherein the updating component further determines whether to update the library datastore with the identifier based also on a second determination resolving a second comparison of consistency of a first content of the identifier and a second content of the library identifier.
Clause 9. A computer-implemented method, comprising:
   identifying, by a system operatively coupled to a processor, chemical compound data describing a chemical compound; and
   generating, by the system, an identifier from the chemical compound data based on an annotation type of the compound data as compared to an annotation ranking schema.
Clause 10. The computer-implemented method of clause 9, further comprising:
   identifying, by the system, plural aspects of the chemical compound data having varying annotation types, including the annotation type; and
   selecting, by the system, a first aspect, of the plural aspects, and having the annotation type, to employ for the identifier based on the annotation type being a highest ranked annotation type, of the varying annotation types, based on the annotation ranking schema.
Clause 11. The computer-implemented method of clause 10, further comprising:
   prioritizing, by the system, the plural aspects according to the varying annotation types as compared to the annotation ranking schema.
Clause 12. The computer-implemented method of clause 10, further comprising:
   generating, by the system, a second identifier based on a lesser ranking second annotation type, of the varying annotation types, as compared to the annotation ranking schema,
   wherein a second content of the second identifier and a content of the identifier consistently describe a same property of the chemical compound.
Clause 13. The computer-implemented method of clause 9, further comprising:
   updating, by the system, a library datastore comprising library identifiers for chemical compounds, including the chemical compound, with the identifier for the chemical compound; and
   directing, by the system, a query corresponding to the chemical compound, to the library datastore, and returns, for the same query, plural identifiers, including the identifier, having been generated for the same chemical compound based on the annotation ranking schema.
Clause 14. The computer-implemented method of clause 9, further comprising:
   comparing, by the system, the identifier and a library identifier for the chemical compound, of a library datastore; and
   determining, by the system, whether to update the library datastore with the identifier based on a first determination resolving a first comparison of the annotation type corresponding to the identifier and a second annotation type corresponding to the library identifier,
   wherein the first comparison comprises a first sub-comparison of forms of the annotation type and the second annotation type, a second sub-comparison of rankings of the annotation type and the second annotation type as compared to the annotation ranking schema, or both.
Clause 15. The computer-implemented method of clause 14, further comprising:
   determining, by the system, whether to update the library datastore with the identifier based also on a second determination resolving a second comparison of consistency of a first content of the identifier and a second content of the library identifier.
Clause 16. A computer program product facilitating a process for generating chemical compound identifiers based on varying annotation types, the computer program product comprising a computer readable storage medium having program instructions embodied therewith, and the program instructions executable by a processor to cause the processor to:
   identify, by the processor, chemical compound data describing a chemical compound; and
   generate, by the processor, an identifier from the chemical compound data based on an annotation type of the compound data as compared to an annotation ranking schema.
Clause 17. The computer program product of clause 16, wherein the program instructions are further executable by the processor to cause the processor to:
   identify, by the processor, plural aspects of the chemical compound data having varying annotation types, including the annotation type; and
   select, by the processor, a first aspect, of the plural aspects, and having the annotation type, to employ for the identifier based on the annotation type being a highest ranked annotation type, of the varying annotation types, based on the annotation ranking schema.
Clause 18. The computer program product of clause 17, wherein the program instructions are further executable by the processor to cause the processor to:
   prioritize, by the processor, the plural aspects according to the varying annotation types as compared to the annotation ranking schema.
Clause 19. The computer program product of clause 17, wherein the program instructions are further executable by the processor to cause the processor to:
   generate, by the processor, a second identifier based on a lesser ranking second annotation type, of the varying annotation types, as compared to the annotation ranking schema,
   wherein a second content of the second identifier and a content of the identifier consistently describe a same property of the chemical compound.
Clause 20. The computer program product of clause 16, wherein the program instructions are further executable by the processor to cause the processor to:
   update, by the processor, a library datastore comprising library identifiers for chemical compounds, including the chemical compound, with the identifier for the chemical compound; and
   direct, by the processor, a query corresponding to the chemical compound, to the library datastore, and returns, for the same query, plural identifiers, including the identifier, having been generated for the same chemical compound based on the annotation ranking schema.

### DETAILED DESCRIPTION

The following detailed description is merely illustrative and is not intended to limit embodiments and/or application or utilization of embodiments. Furthermore, there is no intention to be bound by any expressed or implied information presented in the preceding Summary section, or in the Detailed Description section. One or more embodiments are now described with reference to the drawings, wherein like reference numerals are utilized to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Various operations can be described as multiple discrete actions or operations in turn, in a manner that is most helpful in understanding the subject matter disclosed herein. However, the order of description should not be construed as to imply that these operations are necessarily order dependent. In particular, these operations can be performed in an order different from the order of presentation. Operations described can be performed in a different order from the described embodiment. Various additional operations can be performed, and/or described operations can be omitted in additional embodiments.

Turning now to the subject of library spectral content, such content can be generated, used, searched, etc. for various purposes, not the least of which can comprise discerning identities, similarities, differences and/or relationships corresponding to one or more compounds (and/or spectra of compounds) based on such library of spectra content. For example, this can allow for understanding and/or analysis of mass spectroscopy/mass spectroscopy (MS/MS) data, such as related to fragmentation patterns of chemicals. In existing frameworks, such use of library spectral content comes with a difficulty related to generation and search of metadata employed to identify, label and/or otherwise describe compound data of a library datastore.

For example, with existing frameworks, library data is generated based on a particular annotation type and updated to a library. A library can therefore have varying data based on varying annotation types, and thus have metadata identifiers based on such varying annotation types. These identifiers are not cross-checked with one another for consistency, and thus as a result, a library can have identifiers that correlate to a same compound, but are inconsistent with one another. That is, the content of the annotation types for a same compound can be inconsistent with one another. Indeed, no consideration is given regarding comparison of content to one another based on different annotation types. No consideration is given regarding use of any particular one or more annotation types as being guidelines and/or of highest priority. As a consequence, a search of such library can result in a return of identifiers with conflicting content and thus return of conflicting data.

To account for one or more deficiencies of such existing frameworks, one or more embodiments are described herein that can provide increase of accuracy and efficiency of generation and use of prioritized and cross-checked identifiers. Generally, the one or more embodiments described herein can employ a novel system that provides for generation and use of varying annotation types across different identifiers for same compounds and/or different compounds of a library datastore without consistency issues between the identifiers having been generated by the one or more embodiments described herein. In this way, queries to such library can be returned relative to one or relative to plural annotation types, regardless of an annotation type associated with a query and/or an annotation type associated with library data of the library. Thus, cross-referencing of similarities, differences and/or relationships between compounds (including spectra of the compounds) of such library can be facilitated and executed with ease and efficiency.

That is, the one or more embodiments described herein can provide generation of identifiers using a system to cross-check consistency of content underlying aspects of compound data that are based on different annotation types, an annotation ranking schema to prioritize different annotation types regardless of content, cross-checking of generated identifiers with library identifiers already present in a library for a same compound to determine priority of the identifiers based on the annotation ranking schema and to cross-check the content of theses identifiers for consistency with one another, and use of the annotation ranking schema to resolve one or more consistency conflicts between identifiers.

One or more benefits can comprise an ability to differentiate between such varying annotation types for generating the one or more identifiers, an ability to customize an annotation ranking schema to adjust ranking data employed to provide rankings for the varying annotation types of input compound data (e.g., input to a non-limiting system described herein), and/or application of an embodiment described herein on a plug-and-play basis to various architectures of existing spectral library and/or library datastores of spectral data. That is, the one or more embodiments described herein can generate identifiers for compounds (including for spectra corresponding to compounds) regardless of data structure of a spectral library and/or library datastore. Further, the one or more embodiments described herein can be implemented within, in connection with and/or coupled to a scientific imaging device.

Discussion next turns to a general discussion of one or more scientific instrument systems disclosed herein, as well as to related methods, computing devices, and/or computer-readable media. For example, in one or more embodiments, a system can comprise a memory that stores computer executable components and a processor that executes the computer executable components stored in the memory. The computer executable components can comprise an identifying component that identifies chemical compound data describing a chemical compound; and a generating component that generates an identifier from the chemical compound data based on an annotation type of the compound data as compared to an annotation ranking schema.

The one or more embodiments disclosed herein can achieve improved performance relative to existing approaches, as noted above. For example, based on application of varying annotation types for library spectra content identifier metadata, in combination with various cross-checks of content consistency and use of an annotation ranking schema, varying identifiers can be generated for a same compound that are consistent with one another. That is, the content of the identifiers can be consistent with one another and priority of use of such content can be based on use of an annotation ranking schema. This schema can be employed to determine priority of identifier generation, whether to merge or replace identifiers with existing library identifiers, and/or to resolve conflicts between identifiers.

The embodiments disclosed herein thus can provide improvements to scientific instrument technology (e.g., improvements in the computer technology supporting such scientific instruments, among other improvements), which can be employed for compound analysis in various fields including optics, signal processing, spectroscopy, and/or nuclear magnetic resonance (NMR), without being limited thereto.

Various ones of the embodiments disclosed herein can improve upon existing approaches to achieve the technical advantages of high information and/or accurate information identifier generation and library querying. That is, the one or more embodiments described herein can provide generation of identifiers for compound data based on varying annotation types comprising varying text, code and/or metadata. Indeed, a benefit of the one or more embodiments described herein is an ability to differentiate between such varying annotation types for generating the one or more identifiers. Based thereon, the one or more embodiments described herein can be employed to generate the identifiers such that a library updated with the identifiers can be searched in an annotation type-agnostic manner. That is, information can be returned in response to a query that is based on different annotation types (e.g., plural annotation types) for a same compound. Accordingly, a search employing one annotation type can return data from another annotation type that has already been cross-checked for consistency and/or labeled for ranking based on the annotation ranking schema. These can be useful processes for varying industries employing material analysis, product manufacturing, quality control and/or the like. The embodiments disclosed herein thus can provide improvements to scientific instrument technology (e.g., improvements in the computer technology supporting such scientific instruments, among other improvements).

Such technical advantages are not achievable by routine and/or existing approaches, as described above, and all user entities of systems including such embodiments can benefit from these advantages (e.g., by assisting the user entity in the performance of a technical task, such as return of one or more compound queries, by means of a library datastore, with consistent and ranked identifier metadata.

The technical features of the embodiments disclosed herein (e.g., generation, analysis of, and use of data identifiers using varying annotation types for a library datastore) are thus decidedly unconventional in the field of material analysis, in addition to the fields of optics, signal processing, spectroscopy, and/or NMR, without being limited thereto, as are combinations of the features of the embodiments disclosed herein.

As discussed further herein, various aspects of the embodiments disclosed herein can improve the functionality of a computer itself. That is, the computational and/or user interface features disclosed herein do not involve only the collection and/or comparison of information but instead can apply new analytical and technical techniques to change the operation of the computer-analysis of material compounds. For example, as identifiers are generated for compound data based on varying annotation types and a corresponding annotation ranking schema, comparisons for determining whether or not to update a library can become more efficient and accurate over time. That is, as more library content is added by the embodiments described herein, a larger body of accurate comparative data is generated for use in searches, queries, and/other comparisons performed relative to the various cross-checks employed by the one or more embodiments described herein to generate the identifiers for the spectral content in the first instance. As such, one or more non-limiting systems described herein, comprising an identifier generation system, can be self-improving.

The present disclosure thus introduces functionality that neither an existing computing device, nor a human, could perform. Rather, such existing computing devices are ineffective at analyzing computer data/metadata defining a plurality of compounds, or defining spectra for a plurality of compounds, and/or at generating computer-usable metadata identifiers for a computer-based search of library data stored at a memory device, as the one or more embodiments described herein can provide this process. In view of the time, energy and/or loss of data involved, it is not practical to operate within the confines of existing approaches.

Accordingly, the embodiments of the present disclosure can serve any of a number of technical purposes, such as controlling a specific technical system or process; determining from measurements how to control a machine; digital audio, image, or video enhancement or analysis; separation of material sources in a mixed signal; generating data for reliable and/or efficient transmission or storage; providing estimates and confidence intervals for material samples; or providing a faster processing of sensor data. In particular, the present disclosure provides technical solutions to technical problems, including, but not limited to, hologram modification; image/signal blurring; application of combined blurring techniques; and/or subsequent image reconstruction, resulting in a faster, more thorough and/or more efficient processing of generated images and thus of material samples or other target compositions being imaged.

The embodiments disclosed herein thus provide improvements to material analysis technology (e.g., improvements in the computer technology supporting material analysis, among other improvements).

As used herein, the phrase "based on" should be understood to mean "based at least in part on," unless otherwise specified.

As used herein, the term "component" can refer to an atomic element, molecular element, phase of an atomic or molecular element, or combination thereof.

As used herein, the term "compound" can refer to a single material, multiple materials, composition, sample, solution, product, etc.

As used herein, the term "data" can comprise metadata.

As used herein, the terms "entity," "requesting entity," and "user entity" can refer to a machine, device, component, hardware, software, smart device, party, organization, individual and/or human.

One or more embodiments are now described with reference to the drawings, where like referenced numerals are used to refer to like drawing elements throughout. In the following description, for purposes of explanation, numerous specific details are set forth in order to provide a more thorough understanding of the one or more embodiments. It is evident in various cases, however, that the one or more embodiments can be practiced without these specific details.

Further, it should be appreciated that the embodiments depicted in one or more figures described herein are for illustration only, and as such, the architecture of embodiments is not limited to the systems, devices and/or components depicted therein, nor to any particular order, connection and/or coupling of systems, devices and/or components depicted therein.

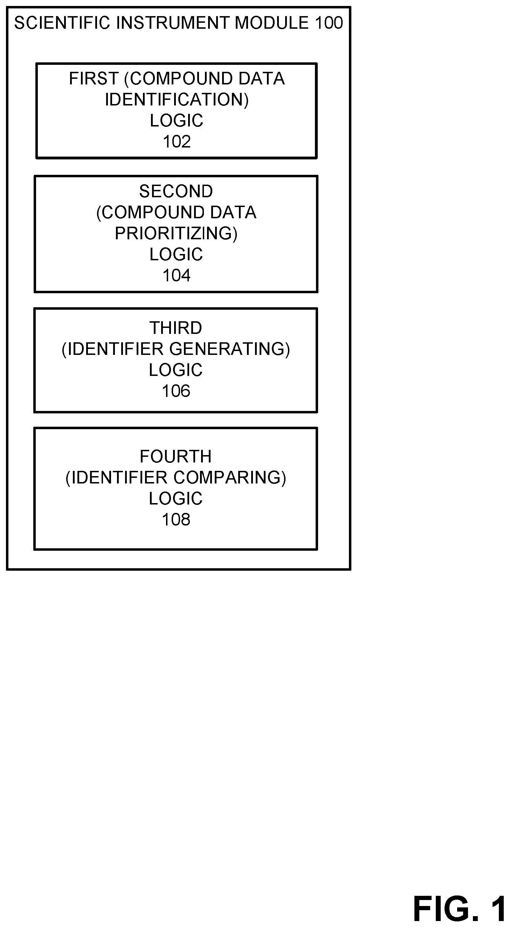

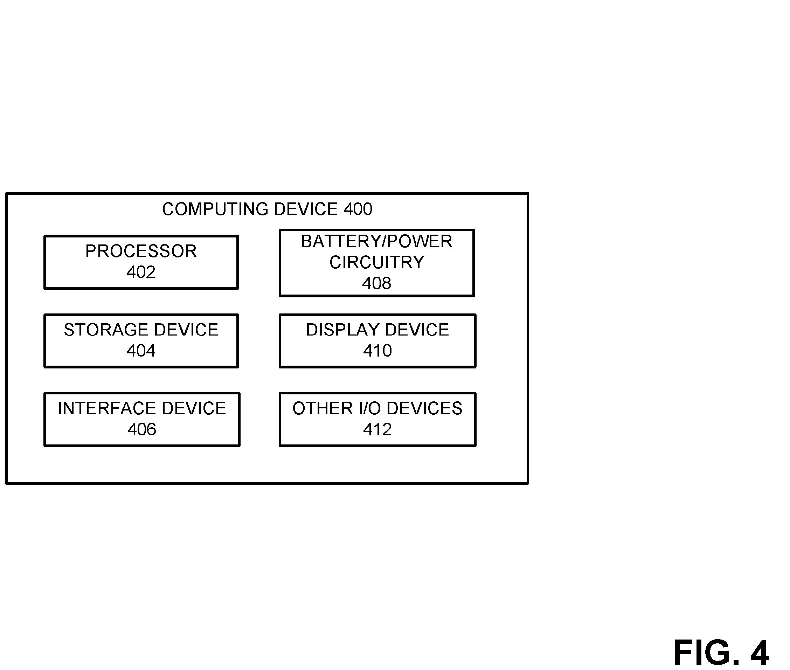

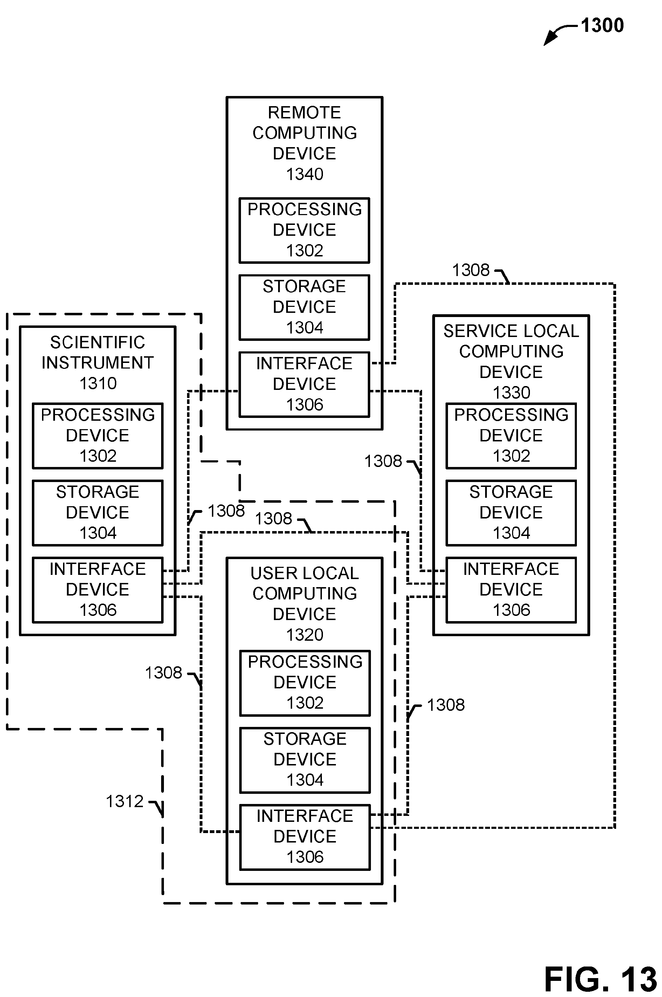

Turning now in particular to the one or more figures, and first to FIG. 1, illustrated is a block diagram of a scientific instrument module 100 for performing material analysis operations using an identifier generation and/or updating process, in accordance with various embodiments described herein. The scientific instrument module 100 can be implemented by circuitry (e.g., including electrical and/or optical components), such as a programmed computing device. The logic of the scientific instrument module 100 can be included in a single computing device or can be distributed across multiple computing devices that are in communication with each other as appropriate. Examples of computing devices that can, singly or in combination, implement the scientific instrument module 100 are discussed herein with reference to the computing device 400 of FIG. 4, and examples of systems of interconnected computing devices, in which the scientific instrument module 100 can be implemented across one or more of the computing devices, is discussed herein with reference to the scientific instrument system 1300 of FIG. 13.

The scientific instrument module 100 can include first logic 102, second logic 104, third logic 106, and fourth logic 108. As used herein, the term "logic" can include an apparatus that is to perform a set of operations associated with the logic. For example, any of the logic elements included in the module 100 can be implemented by one or more computing devices programmed with instructions to cause one or more processing devices of the computing devices to perform the associated set of operations. In a particular embodiment, a logic element can include one or more non-transitory computer-readable media having instructions thereon that, when executed by one or more processing devices of one or more computing devices, cause the one or more computing devices to perform the associated set of operations. As used herein, the term "module" can refer to a collection of one or more logic elements that, together, perform a function associated with the module. Different ones of the logic elements in a module can take the same form or can take different forms. For example, some logic in a module can be implemented by a programmed general-purpose processing device, while other logic in a module can be implemented by an application-specific integrated circuit (ASIC). In another example, different ones of the logic elements in a module can be associated with different sets of instructions executed by one or more processing devices. A module can omit one or more of the logic elements depicted in the associated drawing; for example, a module can include a subset of the logic elements depicted in the associated drawing when that module is to perform a subset of the operations discussed herein with reference to that module.

The first logic 102 can receive, find, locate, download, request, measure and/or otherwise determine chemical compound data and/or annotation types thereof. That is, the first logic 102 can obtain data for being processed and for subsequent use in generating an identifier and/or updating a spectral library.

The second logic 104 can perform a prioritizing process by generally prioritizing varying annotation types of varying aspects of the chemical compound data based on an annotation ranking schema. That is, the second logic 104 can employ the output of the first logic 102 as a trigger for the second logic 104.

The third logic 106 can generate an identifier based on the prioritizing of the second logic 104. That is, the third logic 106 can employ an output of the second logic 104 to perform the third logic 106.

The fourth logic 108 can perform one or more comparisons of the generated identifier and a library identifier to determine how and/or whether to update the library datastore comprising the library identifier. That is, the fourth logic 108 can generate an updating determination based on the execution of the third logic 106.

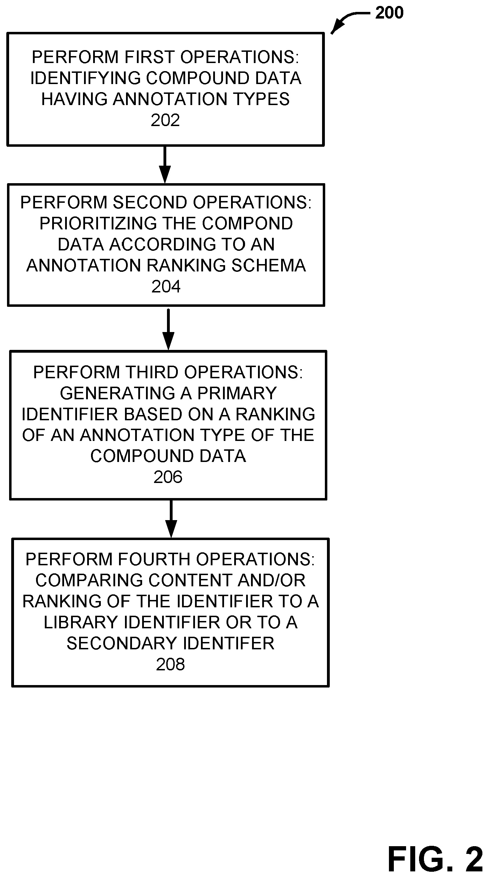

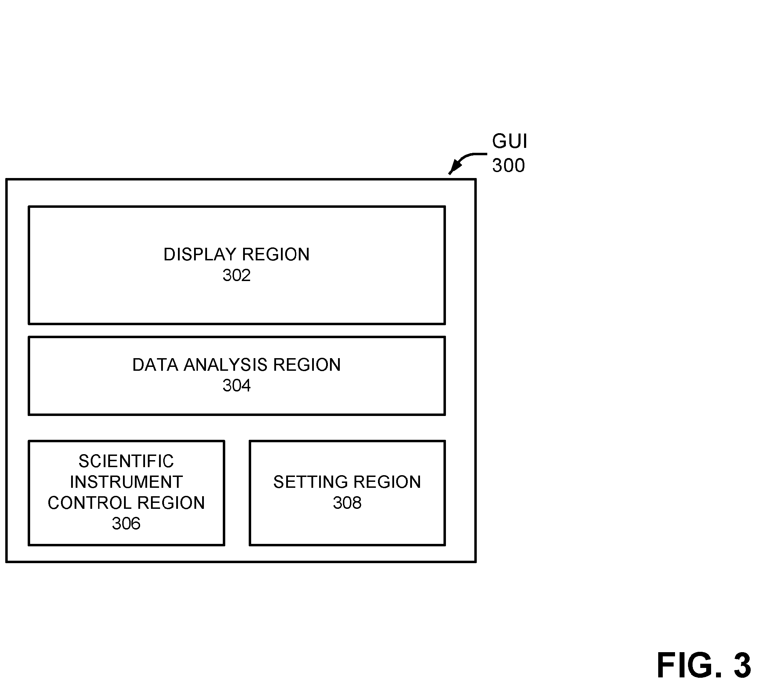

FIG. 2 illustrates a flow diagram of a method 200 of performing operations, by the scientific instrument module 100, in accordance with various embodiments. Although the operations of the method 200 can be illustrated with reference to particular embodiments disclosed herein (e.g., the scientific instrument module 100 discussed herein with reference to FIG. 1, the GUI 300 discussed herein with reference to FIG. 3, the computing device 400 discussed herein with reference to FIG. 4, and/or the scientific instrument system 1300 discussed herein with reference to FIG. 13), the method 200 can be used in any suitable setting to perform any suitable operations. Operations are illustrated once each and in a particular order in FIG. 2, but the operations can be reordered and/or repeated as desired and appropriate (e.g., different operations performed can be performed in parallel, as suitable).

At 202, first operations can be performed. For example, the first logic 102 of the module 100 can perform the first operations 202. The first operations 202 can include receiving, finding, locating, downloading, requesting, measuring and/or otherwise determining chemical compound data and/or annotation types thereof.

At 204, second operations can be performed. For example, the second logic 104 of the module 100 can perform the second operations 204. The second operations 204 can comprise comparing one or more annotation types of the chemical compound data identified to an annotation ranking schema comprising ranking data for the varying annotation types.

At 206, third operations can be performed. For example, the third logic 106 of the module 100 can perform the third operations 206. The third operations 206 can comprise generating an identifier based on the comparing of the second operations 204.

At 208, fourth operations can be performed. For example, the fourth logic 108 of the module 100 can perform the fourth operations 208. The fourth operations 208 can comprise execution of one or more comparisons between the generated identifier and one or more library identifiers 637 at a library datastore that is desired to be updated using and/or with the chemical compound data.

The scientific instrument methods disclosed herein can include interactions with a user entity (e.g., via the user local computing device 1320 discussed herein with reference to FIG. 13). These interactions can include providing information to the user entity (e.g., information regarding the operation of a scientific instrument such as the scientific instrument 1310 of FIG. 13, information regarding a sample being analyzed or other test or measurement performed by a scientific instrument, information retrieved from a local or remote database, or other information) or providing an option for a user entity to input commands (e.g., to control the operation of a scientific instrument such as the scientific instrument 1310 of FIG. 13, or to control the analysis of data generated by a scientific instrument), queries (e.g., to a local or remote database), or other information. In some embodiments, these interactions can be performed through a graphical user interface (GUI) that includes a visual display on a display device (e.g., the display device 410 discussed herein with reference to FIG. 4) that provides outputs to the user entity and/or prompts the user entity to provide inputs (e.g., via one or more input devices, such as a keyboard, mouse, trackpad, or touchscreen, included in the other I/O devices 412 discussed herein with reference to FIG. 4). The scientific instrument system 1300 disclosed herein can include any suitable GUIs for interaction with a user entity.

Turning next to FIG. 3, depicted is an example GUI 300 that can be used in the performance of one or more of the methods described herein, in accordance with various embodiments described herein. As noted above, the GUI 300 can be provided on a display device (e.g., the display device 410 discussed herein with reference to FIG. 4) of a computing device (e.g., the computing device 400 discussed herein with reference to FIG. 4) of a scientific instrument system (e.g., the scientific instrument system 1300 discussed herein with reference to FIG. 13), and a user entity can interact with the GUI 300 using any suitable input device (e.g., any of the input devices included in the other I/O devices 412 discussed herein with reference to FIG. 4) and input technique (e.g., movement of a cursor, motion capture, facial recognition, gesture detection, voice recognition, actuation of buttons, etc.).

The GUI 300 can include a data display region 302, a data analysis region 304, a scientific instrument control region 306, and a settings region 308. The particular number and arrangement of regions depicted in FIG. 3 is merely illustrative, and any number and arrangement of regions, including any desired features thereof, can be included in a GUI 300.

The data display region 302 can display data generated by a scientific instrument (e.g., the scientific instrument 1310 discussed herein with reference to FIG. 13). For example, the data display region 302 can display one or more output results which can comprise one or more spectra, one or more annotation rankings, one or more aspects of the chemical compound data, a visualization of an annotation ranking schema, etc., without being limited thereto.

The data analysis region 304 can display the results of data analysis (e.g., the results of analyzing the data illustrated in the data display region 302 and/or other data). For example, the data analysis region 304 can display one or more of the output results of a query (e.g., a chemical compound), such as a classification defining the chemical compound. In one or more cases, the data analysis region 304 can display a list, flow chart or other schematic of acquisition actions taken and/or recommended relative to an experiment. In one or more embodiments, the data display region 302 and the data analysis region 304 can be combined in the GUI 300 (e.g., to include data output from a scientific instrument, and some analysis of the data, in a common graph or region).

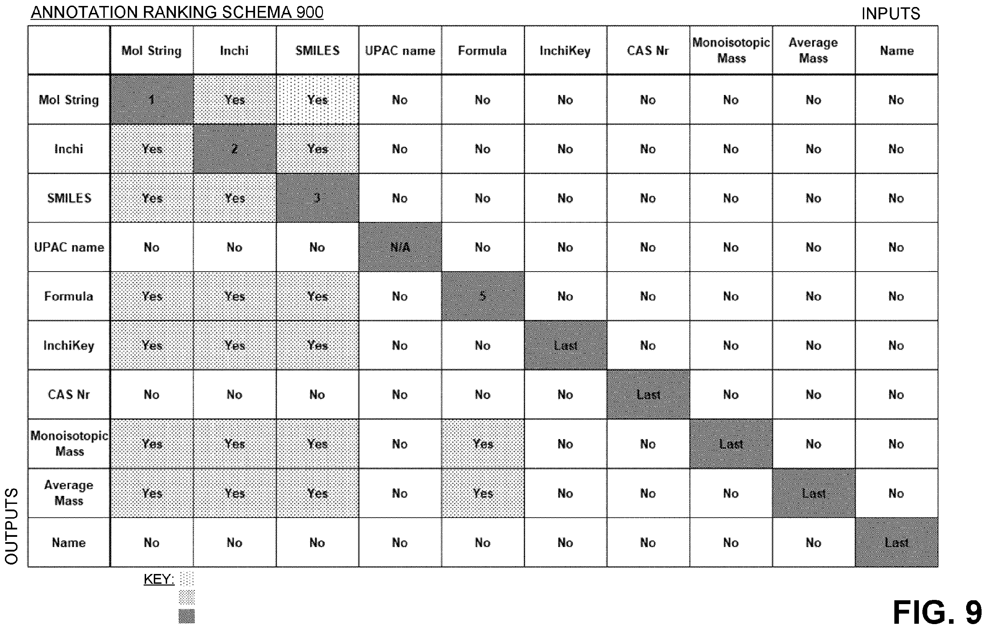

The scientific instrument control region 306 can include options that allow the user entity to control a scientific instrument (e.g., the scientific instrument 1310 discussed herein with reference to FIG. 13). For example, the scientific instrument control region 306 can include one or more controls for customizing a cloud visual, such as based on the GUI 900 of FIG. 9, to be described below.

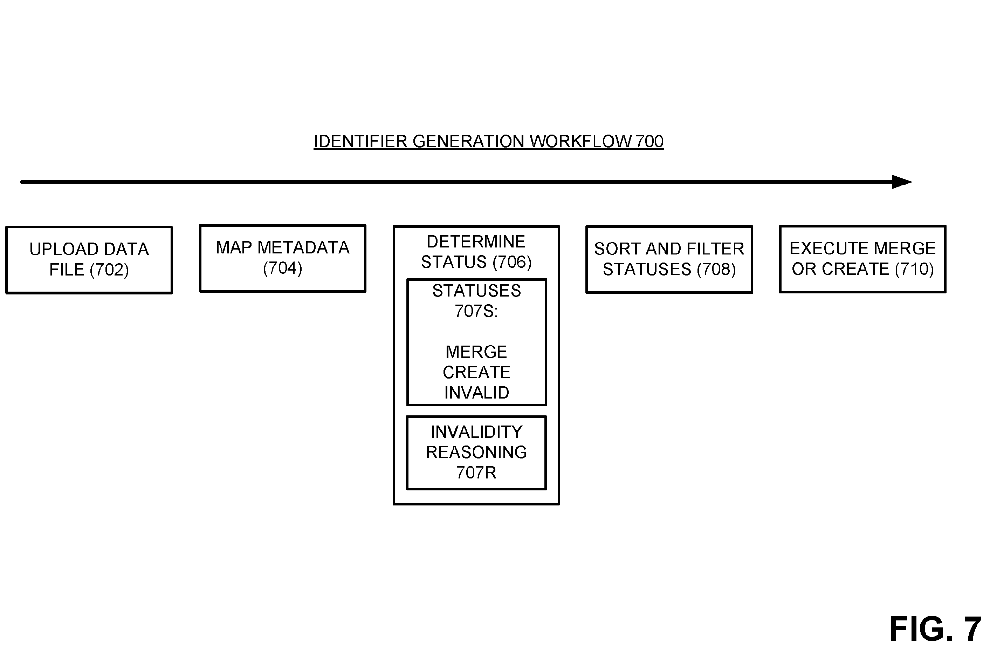

The settings region 308 can include options that allow the user entity to control the features and functions of the GUI 300 (and/or other GUIs) and/or perform common computing operations with respect to the data display region 302 and data analysis region 304 (e.g., saving data on a storage device, such as the storage device 404 discussed herein with reference to FIG. 4, sending data to another user entity, labeling data, etc.). For example, the settings region 308 can include one or more options to alter color, fill or format of illustrations, such as an illustration of any aspect of FIGS. 7-9 and/or other image, whether actual, representative and/or schematic, to be described below.

As noted above, the scientific instrument module 100 can be implemented by one or more computing devices. Accordingly, discussion next turns to FIG. 4, which illustrates a block diagram of a computing device 400 that can perform some or all of the scientific instrument methods disclosed herein, in accordance with various embodiments. In one or more embodiments, the scientific instrument module 100 can be implemented by a single computing device 400 or by multiple computing devices 400. Further, as discussed below, a computing device 400 (or multiple computing devices 400) that implements the scientific instrument module 100 can be part of one or more of the scientific instrument 1310, the user local computing device 1320, the service local computing device 1330, or the remote computing device 1340 of FIG. 13.

The computing device 400 of FIG. 4 is illustrated as having a number of components, but any one or more of these components can be omitted or duplicated, as suitable for the application and setting. As illustrated, these components can include one or more of a processor 402, storage device 404, interface device 406, battery/power circuitry 408, display device 410 and other input/output (I/O) devices 412, as will be described below.

In one or more embodiments, one or more of the components included in the computing device 400 can be attached to one or more motherboards and enclosed in a housing (e.g., including plastic, metal, and/or other materials). In one or more embodiments, some these components can be fabricated onto a single system-on-a-chip (SoC) (e.g., an SoC can include one or more processors 402 and one or more storage devices 404). Additionally, in one or more embodiments, the computing device 400 can omit one or more of the components illustrated in FIG. 4. In one or more embodiments, the computing device 400 can include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface). For example, the computing device 400 can omit a display device 410, but can include display device interface circuitry (e.g., a connector and driver circuitry) to which a display device 410 can be coupled.

The computing device 400 can include the processor 402 (e.g., one or more processing devices). As used herein, the term "processing device" can refer to any device or portion of a device that processes electronic data from registers and/or memory to transform that electronic data into other electronic data that can be stored in registers and/or memory. The processor 402 can include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 400 can include a storage device 404 (e.g., one or more storage devices). The storage device 404 can include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In one or more embodiments, the storage device 404 can include memory that shares a die with a processor 402. In such an embodiment, the memory can be used as cache memory and can include embedded dynamic random-access memory (eDRAM) or spin transfer torque magnetic random-access memory (STT-MRAM), for example. In one or more embodiments, the storage device 404 can include non-transitory computer readable media having instructions thereon that, when executed by one or more processing devices (e.g., the processor 402), cause the computing device 400 to perform any appropriate ones of or portions of the methods disclosed herein.

The computing device 400 can include an interface device 406 (e.g., one or more interface devices 406). The interface device 406 can include one or more communication chips, connectors, and/or other hardware and software to govern communications between the computing device 400 and other computing devices. For example, the interface device 406 can include circuitry for managing wireless communications for the transfer of data to and from the computing device 400. The term "wireless" and its derivatives can be used to describe circuits, devices, systems, methods, techniques, communications channels, etc., that can communicate data through the use of modulated electromagnetic radiation through a nonsolid medium. The term does not imply that the associated devices do not contain any wires, although in one or more embodiments the associated devices might not contain any wires. Circuitry included in the interface device 406 for managing wireless communications can implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In one or more embodiments, circuitry included in the interface device 406 for managing wireless communications can operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In one or more embodiments, circuitry included in the interface device 406 for managing wireless communications can operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In one or more embodiments, circuitry included in the interface device 406 for managing wireless communications can operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In one or more embodiments, the interface device 406 can include one or more antennas (e.g., one or more antenna arrays) to receipt and/or transmission of wireless communications.

In one or more embodiments, the interface device 406 can include circuitry for managing wired communications, such as electrical, optical, or any other suitable communication protocols. For example, the interface device 406 can include circuitry to support communications in accordance with Ethernet technologies. In one or more embodiments, the interface device 406 can support both wireless and wired communication, and/or can support multiple wired communication protocols and/or multiple wireless communication protocols. For example, a first set of circuitry of the interface device 406 can be dedicated to shorter-range wireless communications such as Wi-Fi or Bluetooth, and a second set of circuitry of the interface device 406 can be dedicated to longer-range wireless communications such as global positioning system (GPS), EDGE, GPRS, CDMA, WiMAX, LTE, EV-DO, or others. In one or more embodiments, a first set of circuitry of the interface device 406 can be dedicated to wireless communications, and a second set of circuitry of the interface device 406 can be dedicated to wired communications.

The computing device 400 can include battery/power circuitry 408. The battery/power circuitry 408 can include one or more energy storage devices (e.g., batteries or capacitors) and/or circuitry for coupling components of the computing device 400 to an energy source separate from the computing device 400 (e.g., AC line power).

The computing device 400 can include a display device 410 (e.g., multiple display devices). The display device 410 can include any visual indicators, such as a heads-up display, a computer monitor, a projector, a touchscreen display, a liquid crystal display (LCD), a light-emitting diode display, or a flat panel display.

The computing device 400 can include other input/output (I/O) devices 412. The other I/O devices 412 can include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 400, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 400 can have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

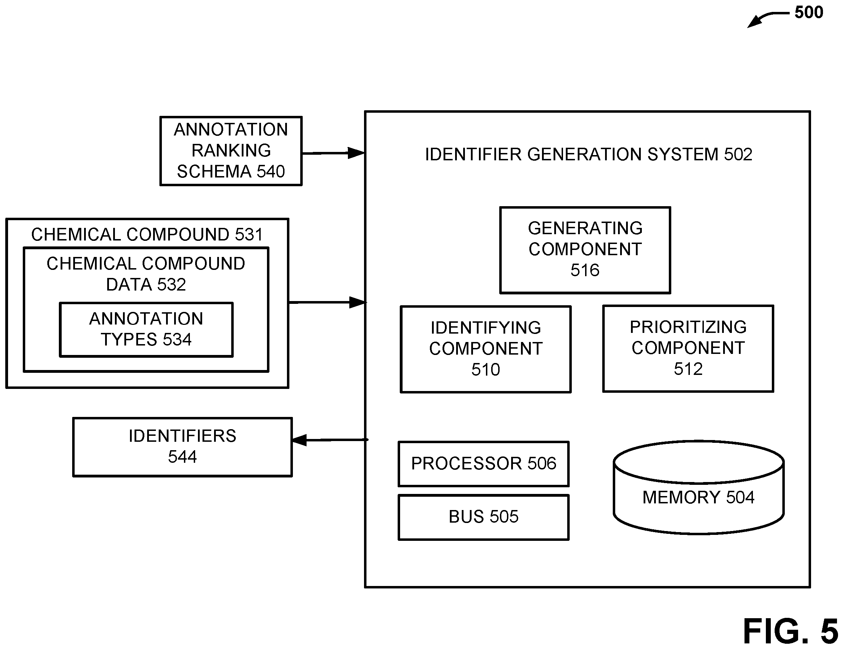

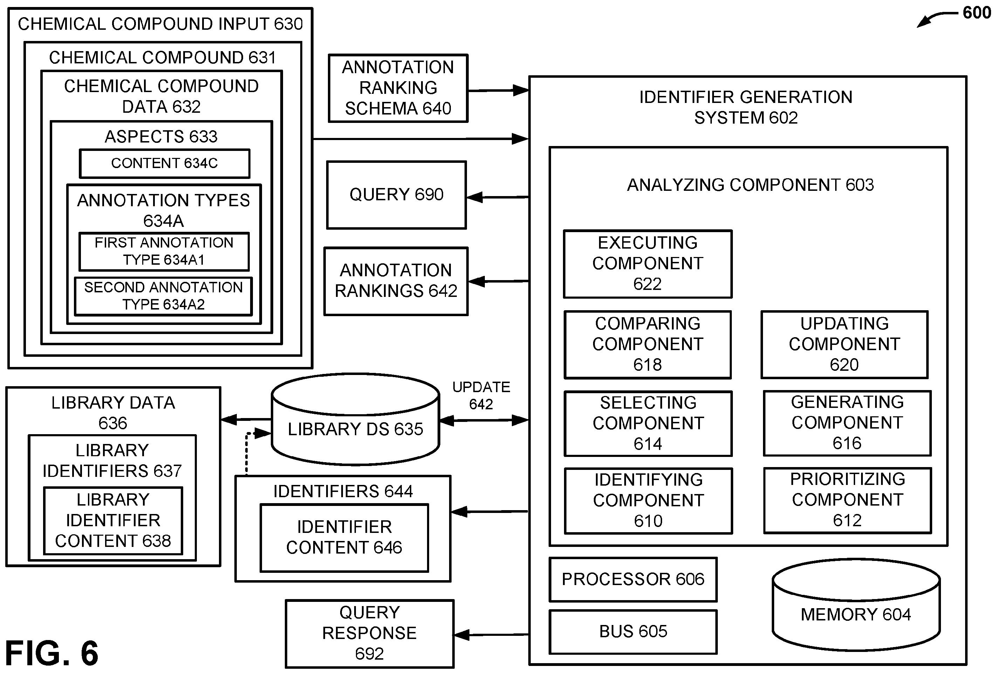

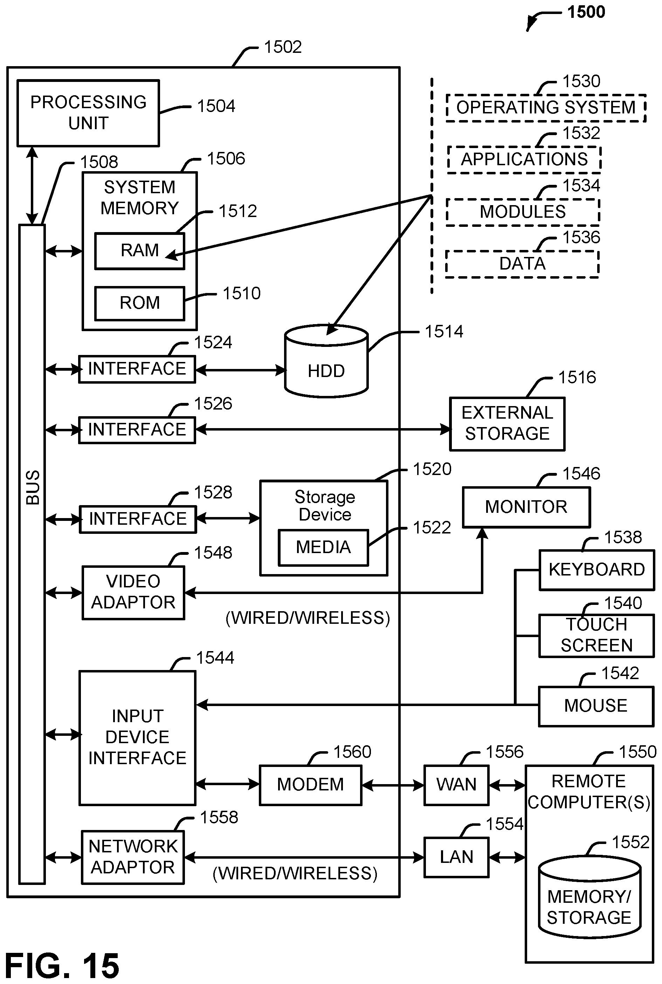

Referring now to FIGS. 5 and 6, in one or more embodiments, the non-limiting systems 500 and/or 600 illustrated at FIGS. 5 and 6, and/or systems thereof, can further comprise one or more computer and/or computing-based elements described herein with reference to a computing environment, such as the computing environment 1500 illustrated at FIG. 15. In one or more described embodiments, computer and/or computing-based elements can be used in connection with implementing one or more of the systems, devices, components and/or computer-implemented operations shown and/or described in connection with FIGS. 5 and/or 6 and/or with other figures described herein.

Turning first to FIG. 5, the figure illustrates a block diagram of an example, non-limiting system 500 that can comprise an identifier generation system 502. The identifier generation system 502 can generally facilitate generation of an identifier comprising metadata for describing compound data and which is based on one of varying annotation types that can be employed by identifiers of a library datastore.

In one or more embodiments, the identifier generation system 502 can be at least partially comprised by the computing device 400.

It is noted that the identifier generation system 502 is only briefly detailed to provide but a lead-in to a more complex and/or more expansive identifier generation system 602 as illustrated at FIG. 6. That is, further detail regarding processes that can be performed by one or more embodiments described herein will be provided below relative to the non-limiting system 600 of FIG. 6.

Still referring to FIG. 5, the identifier generation system 502 can comprise at least a memory 504, bus 505, processor 506, identifying component 510, prioritizing component 512 and/or generating component 516. The processor 506 can be the same as the processor 402, comprised by the processor 402 or different therefrom. The memory 504 can be the same as the storage device 404, comprised by the storage device 404 or different therefrom.

Using the above-noted components, the identifier generation system 502 can facilitate a process to determine annotation type of input compound data, prioritize the annotation type based on an annotation ranking schema, and employ the compound data and the annotation type to generate an identifier based on the annotation ranking schema results.

Generally, the identifying component 510 can identify chemical compound data 532 describing a chemical compound 531 and being based on a particular annotation type 534. The annotation type can be one of varying annotation types that are typically employed by a user entity and/or that are employed by a library datastore for which it is desired that the compound data 532 be updated into the library datastore and/or cross-referenced with library data of the library datastore.

The prioritizing component 512 can generally determine whether the annotation type 534 of the compound data 532 correlates with an annotation type of a specified annotation ranking schema 540. The annotation ranking schema 540 can be employed to determine priority of one annotation type as compared to a plurality of one or more other annotation types 534.

The generating component 516 generally can generate an identifier 544 from the chemical compound data 532 based on an annotation type 534 of the compound data 532 as compared to the annotation ranking schema 540. The identifier 544, and/or metadata thereof, can be employed to describe different chemical compound data 532, for identifying such chemical compound data 532 in response to a query, and/or for categorically and/or hierarchically storing such chemical compound data 532 at a library datastore, without being limited thereto.

As a result of these components, the content of the identifiers 544 at a same library datastore can be consistent with one another and priority of use of such content can be based on use of an annotation ranking schema 540. This schema 540 can be employed to determine priority of identifier generation, whether to merge or replace identifiers with existing library identifiers, and/or to resolve conflicts between identifiers.

The identifying component 510, prioritizing component 512 and/or generating component 516 can be operatively coupled to the processor 506 which can be operatively coupled to the memory 504. The bus 505 can provide for the operative coupling. The processor 506 can facilitate execution of the identifying component 510, prioritizing component 512 and/or generating component 516. The identifying component 510, prioritizing component 512 and/or generating component 516 can be stored at the memory 504.

In general, the non-limiting system 500 can employ any suitable method of communication (e.g., electronic, communicative, internet, infrared, fiber, etc.) to provide communication between the identifier generation system 502, a library datastore, and/or any device associated with a user entity.

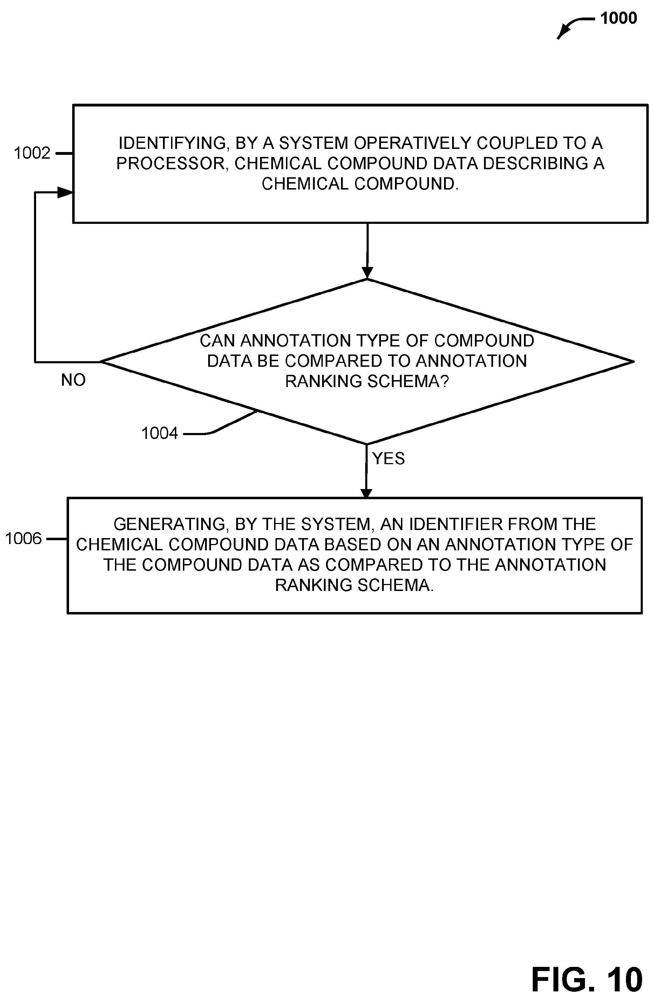

As a summary of the above-described components and functions thereof, referring next only briefly to FIG. 10, illustrated is a flow diagram of an example, non-limiting method 1000 that can facilitate a process for identifier generation and subsequent updating of library content based on the identifier. While the non-limiting method 1000 is described relative to the non-limiting system 500 of FIG. 5, the non-limiting method 1000 can be applicable also to other systems described herein, such as the non-limiting system 600 of FIG. 6. Repetitive description of like elements and/or processes employed in respective embodiments is omitted for sake of brevity.

At 1002, the non-limiting method 1000 can comprise identifying, by the system (e.g., identifying component 510) operatively coupled to a processor, chemical compound data (e.g., chemical compound data 532) describing a chemical compound (e.g., chemical compound 531).

At 1004, the non-limiting method 1000 can comprise determining, by the system (e.g., prioritizing component 512), whether an annotation type (e.g., annotation type 534) of the chemical compound data is comprised by and/or can be compared to a specified annotation ranking schema (e.g., annotation ranking schema 540). If not, the non-limiting method 1100 can proceed back to step 1002. If yes, the non-limiting method can proceed forward to step 1006.

At 1006, the non-limiting method 1000 can comprise generating, by the system (e.g., generating component 516) an identifier (e.g., identifier 544) based on the annotation type of the compound data as compared to the annotation ranking schema..

Turning next to FIG. 6, a non-limiting system 600 is illustrated that can comprise an identifier generation system 602 and a library datastore (DS) 635. Repetitive description of like elements and/or processes employed in respective embodiments is omitted for sake of brevity. Description relative to an embodiment of FIG. 5 can be applicable to an embodiment of FIG. 6. Likewise, description relative to an embodiment of FIG. 6 can be applicable to an embodiment of FIG. 5.

Generally, the identifier generation system 602 can facilitate generation of an identifier 644 comprising metadata for describing compound data 632 and which is based on one of varying annotation types 634A (e.g., categories, elements, etc.) that can be employed by identifiers 637 of a library datastore 635.

In one or more embodiments, the identifier generation system 602 can be at least partially comprised by the computing device 400.

One or more communications between one or more components of the non-limiting system 600 can be provided by wired and/or wireless means including, but not limited to, employing a cellular network, a wide area network (WAN) (e.g., the Internet), and/or a local area network (LAN). Suitable wired or wireless technologies for supporting the communications can include, without being limited to, wireless fidelity (Wi-Fi), global system for mobile communications (GSM), universal mobile telecommunications system (UMTS), worldwide interoperability for microwave access (WiMAX), enhanced general packet radio service (enhanced GPRS), third generation partnership project (3GPP) long term evolution (LTE), third generation partnership project 2 (3GPP2) ultra-mobile broadband (UMB), high speed packet access (HSPA), Zigbee and other 802.XX wireless technologies and/or legacy telecommunication technologies, BLUETOOTH^{®}, Session Initiation Protocol (SIP), ZIGBEE^{®}, RF4CE protocol, WirelessHART protocol, 6LoWPAN (lpv6 over Low power Wireless Area Networks), Z-Wave, an advanced and/or adaptive network technology (ANT), an ultra-wideband (UWB) standard protocol and/or other proprietary and/or non-proprietary communication protocols.

The identifier generation system 602 can be associated with, such as accessible via, a cloud computing environment, such as the cloud computing environment 1500 of FIG. 15.

The identifier generation system 602 can comprise a plurality of components. The components can comprise a memory 604, processor 606, bus 605, identifying component 610, prioritizing component 612, selecting component 614, generating 616, comparing component 618, updating component 620 and/or executing component 622. Using these components, the identifier generation system 602 can generate an identifier 644 based on varying annotation types 634A, determine whether or not to merge or update the identifier 644 into a library datastore 635, and/or resolve a conflict between identifiers 644, 637 based on the annotation ranking schema 640.

Discussion next turns to the processor 606, memory 604 and bus 605 of the identifier generation system 602. For example, in one or more embodiments, the identifier generation system 602 can comprise the processor 606 (e.g., computer processing unit, microprocessor, classical processor, and/or like processor). In one or more embodiments, a component associated with identifier generation system 602, as described herein with or without reference to the one or more figures of the one or more embodiments, can comprise one or more computer and/or machine readable, writable and/or executable components and/or instructions that can be executed by processor 606 to provide performance of one or more processes defined by such component and/or instruction. In one or more embodiments, the processor 606 can comprise the identifying component 610, prioritizing component 612, selecting component 614, generating 616, comparing component 618, updating component 620 and/or executing component 622.

In one or more embodiments, the identifier generation system 602 can comprise the computer-readable memory 604 that can be operably connected to the processor 606. The memory 604 can store computer-executable instructions that, upon execution by the processor 606, can cause the processor 606 and/or one or more other components of the identifier generation system 602 (e.g., identifying component 610, prioritizing component 612, selecting component 614, generating 616, comparing component 618, updating component 620 and/or executing component 622) to perform one or more actions. In one or more embodiments, the memory 604 can store computer-executable components (e.g., identifying component 610, prioritizing component 612, selecting component 614, generating 616, comparing component 618, updating component 620 and/or executing component 622).

The identifier generation system 602 and/or a component thereof as described herein, can be communicatively, electrically, operatively, optically and/or otherwise coupled to one another via a bus 605. Bus 605 can comprise one or more of a memory bus, memory controller, peripheral bus, external bus, local bus, and/or another type of bus that can employ one or more bus architectures. One or more of these examples of bus 605 can be employed.

In one or more embodiments, the identifier generation system 602 can be coupled (e.g., communicatively, electrically, operatively, optically and/or like function) to one or more external systems (e.g., a non-illustrated electrical output production system, one or more output targets and/or an output target controller), sources and/or devices (e.g., computing devices, communication devices and/or like devices), such as via a network. In one or more embodiments, one or more of the components of the identifier generation system 602 and/or of the non-limiting system 600 can reside in the cloud, and/or can reside locally in a local computing environment (e.g., at a specified location).

In addition to the processor 606 and/or memory 604 described above, the identifier generation system 602 can comprise one or more computer and/or machine readable, writable and/or executable components and/or instructions that, when executed by processor 606, can provide performance of one or more operations defined by such component and/or instruction.

Discussion next turns to the additional components of the identifier generation system 602 (e.g., identifying component 610, prioritizing component 612, selecting component 614, generating 616, comparing component 618, updating component 620 and/or executing component 622). Generally, the identifier generation system 602 can perform a set of processes that can be separated into various steps comprising, but not limited to: generation of an identifier 644 based on varying annotation types 634A, determination of whether or not to merge or update the identifier 644 into a library datastore 635, and/or resolution of a conflict between identifiers 644, 637 based on the annotation ranking schema 640.

First, it is noted that in one or more embodiments, the identifying component 610, prioritizing component 612, selecting component 614, generating 616, comparing component 618, updating component 620 and/or executing component 622 can be implemented independently, without one or more other of the identifying component 610, prioritizing component 612, selecting component 614, generating 616, comparing component 618, updating component 620 and/or executing component 622. Additionally and/or alternatively, the identifying component 610, prioritizing component 612, selecting component 614, generating 616, comparing component 618, updating component 620 and/or executing component 622 can be comprised by a high-level analyzing component 603, one or more of the below-described functions of the identifying component 610, prioritizing component 612, selecting component 614, generating 616, comparing component 618, updating component 620 and/or executing component 622 can be performed by the high-level analyzing component 603, and/or the identifying component 610, prioritizing component 612, selecting component 614, generating 616, comparing component 618, updating component 620 and/or executing component 622 can be omitted with the high-level analyzing component 603 performing one or more of the below-described functions of the one or more omitted identifying component 610, prioritizing component 612, selecting component 614, generating 616, comparing component 618, updating component 620 and/or executing component 622.

Turning first to the identifying component 610, this component can generally acquire (e.g., obtain, locate, identify, request, download, etc.) chemical compound data 632 describing a chemical compound 631 and being based on a particular annotation type 634A. The annotation type 634A can be one of varying annotation types 634A that are typically employed by a user entity and/or that are employed by a library datastore 635 for which it is desired that the compound data 532 be updated into the library datastore 635 and/or cross-referenced with library data 636 of the library datastore 635.

That is, the chemical compound data 632 can be based on and/or comprised by a chemical compound input 630 to the identifier generation system 602. Such chemical compound input 630 can comprise any suitable format, text and/or code. The chemical compound input 630 can comprise a request to generate an identifier 644, update a library datastore 635 with the compound data 632, and/or query a library datastore 635.

Such library datastore 635 can be disposed at any suitable location, such as internal to and/or external to the identifier generation system 602 and/or non-limiting system 600. The library datastore 635 can be communicatively couplable to the non-limiting system 600. The library datastore 635 can comprise library data 636 employing metadata of library identifiers 637 that comprise library identifier content 638. The library identifiers 637 can be based on any suitable annotation type, such as a set of varying annotation types such as, but not limited to Mol String, International Chemical Identifier (Inchi), simplified molecular-input line-entry system (SMILES), PubChem, Union of Pure and Applied Chemistry (UPAC) name, formula, InchiKey, Chemical Abstracts Service (CAS), monoisotopic mass, and/or average mass. The library datastore 635 can employ any suitable data, metadata, text, code, etc. and any suitable method of organization of the data stored therein and/or thereat.

Turning now to FIG. 7, in addition to still referring to FIG. 6, after uploading of a chemical compound input 630 (at step 702 of the identifier generation workflow 700) comprising chemical compound data 632, the identifying component 610 can determine an annotation type 634A of the chemical compound data 632 based on historical data, metadata of the chemical compound data 632 and/or comparison to one or more annotation type descriptions. In one or more cases, this can comprise determining various aspects 633 of the chemical compound data 632 as having varying different annotation types 634A, such as a plurality of different annotation types 634A, as noted above (e.g., Mol String, Inchi, SMILES, UPAC name, formula, InchiKey, CAS, monoisotopic mass, and/or average mass. That is, the identifying component 510 can generally map the chemical compound data 632 based on metadata thereof corresponding to the various annotation types (e.g., step 704 of the identifier generation workflow 700).

The prioritizing component 612 can generally determine whether the annotation type 634 of the compound data 632 correlates with an annotation type of a specified annotation ranking schema 640. The annotation ranking schema 640 can be employed to determine priority of one annotation type 634A as compared to a plurality of one or more other annotation types 634A. For example, a compound data 632 can comprise a pair of aspects 633 each based on a different annotation type 634A1 and 634A2. These annotation types 634A1 and 634A2 can be compared to an annotation ranking schema 900 as illustrated at FIG. 9, or to any other suitable annotation ranking schema 640. Based on a format of the annotation types 634A, previous identification of the annotation types 634A and/or metadata thereof, the prioritizing component 612 can generate annotation rankings 642 to hierarchically prioritize the annotation types 634A1 and 634A2.

For example, referring briefly to the annotation ranking schema 900 as illustrated at FIG. 9, a Mol String annotation type can be ranked higher than an Inchi annotation type or than a SMILES annotation type. In another example, an Inchi annotation type 634A2 can be ranked higher than a CAS annotation type 634A1.

It is noted that the annotation ranking schema 640 can be specified by, uploaded by, downloaded by, retrieved by and/or customized by any suitable administrator entity using any suitable computing device that can be communicatively coupled to the non-limiting system 600. In one or more cases, the annotation ranking schema can be customized, in this way, at any suitable time to adjust ranking data underlying the schema 640 that employed to provide annotation rankings 642 for the varying annotation types 634A of the input compound data 632, among other uses.

Based on the determination of the annotation rankings 642, the selecting component 614 can determine a first aspect 633, of the plural aspects 633, to employ for a primary identifier 644 to be generated, based on the respective annotation type 634A thereof being a highest ranked annotation type 634A, of the varying annotation types 634A, based on the annotation ranking schema 640.

The generating component 616 generally can generate an identifier 644 from the chemical compound data 632 based on an annotation type 634 of the compound data 632 as compared to the annotation ranking schema 640. The identifier 644, and/or metadata thereof, can be employed to describe different chemical compound data 632, for identifying such chemical compound data 632 in response to a query, and/or for categorically and/or hierarchically storing such chemical compound data 632 at a library datastore (e.g., library datastore 635), without being limited thereto.

After generation of the primary identifier 644, and/or at least partially in parallel therewith, one or more other secondary identifiers 644 can be generated by the generating component 616, based on other aspects 633 of the compound data 632 for the same compound 631, where these secondary identifiers 644 can be based on different annotation types 634A, other than the annotation type 634A of the primary identifier 644.

In one or more cases, prior to generation of the secondary identifiers, or prior to generation of the primary identifier 644, or at least partially in parallel with either process thereof, the prioritizing component 612 can perform a cross-check of content 634C associated with the aspects 633 to further guide the generating that is performed by the generating component 616. For example, the prioritizing component 612 can compare the content 634C of (e.g., comprised by, associated therewith and/or corresponding to) one aspect 633 to the content 634C of another aspect 633. The contents 634C can comprise metadata describing the chemical compound 631. In one or more cases, such contents 634C can be consistent with one another. That is, the contents 634C can describe same and/or different properties and/or other aspects of a chemical compound 631 in a same or different ways. In one or more other cases, such contents 634C can be inconsistent with one another. That is, the contents 634C can describe same properties and/or other aspects of a chemical compound 631 in conflicting ways, such as where one content 634C is more factually accurate, theoretically accurate, etc. than another content 634C.

In one or more embodiments, such determination can be made based on historical data, a datastore of chemical compound information and/or input to the non-limiting system 600 by a user entity using any suitable computing device communicatively couplable to the non-limiting system 600.

Where contents 634C are consistent, the identifier generation system 602 can proceed to generate primary and secondary identifiers 644 for the respective aspects 633 based on the annotation ranking schema 640, as described above and/or below.

Where contents 634C are inconsistent, the identifier generation system 602 can proceed to generate primary and secondary identifiers 644 for only some of the respective aspects 633 based on the annotation ranking schema 640, as described above and/or below. That is, the annotation ranking schema 640 can be employed to resolve the conflict of consistency, wherein a content 634C associated with a higher annotation type 634A (according to the annotation ranking schema 640) can be maintained and another content 634C associated with a lower annotation type 634A will not be used to generate any identifier 644. That is, the prioritizing component 612 can generate such determination. In this way, the annotation ranking schema 640 can be employed to determine that the content 634C associated with the higher ranking 642 as being consistent and the content 634C associated with the lower ranking 642 as being inconsistent.

In one or more embodiments, such determination can be made not based on the content directly, but rather employing the annotation ranking schema 640.

That is, the annotation ranking schema 640 can be employed to more directly resolve the conflict of consistency, wherein a content 634C associated with a higher annotation type 634A (according to the annotation ranking schema 640) can be maintained and another content 634C associated with a lower annotation type 634A will not be used to generate any identifier 644. That is, the prioritizing component 612 can generate such determination. In this way, the annotation ranking schema 640 can be employed to determine that the content 634C associated with the higher ranking 642 as being consistent and the content 634C associated with the lower ranking 642 as being inconsistent.

Turning again to the generating component 616, the generating performed can comprise writing data and/or metadata to one or more files associated with the chemical compound data aspect 633 to which the annotation type 634 corresponds. The identifier 644 generally can comprise metadata that labels the aspect 633 as corresponding to an annotation type 634A and/or ranking 642. Accordingly, when a system, such as a processor associated with the library datastore 635, searched data comprising the identifier 644, the metadata of the identifier 644 can be employed to properly read the aspect 633 according to the annotation type 634A. Likewise, the metadata of the identifier 644 can be employed to determine priority of data returned based on the ranking 642, as compared to a ranking 642 of another aspect 633.

As will now be described, the metadata of the identifier 644, generated by the non-limiting system 600, further can be employed regardless of the annotation type 634A, based on a set of varying cross-checks and/or validations that can be performed by the identifier generation system 602.

For example, at step 706 of the identifier generation workflow 700, the comparing component 618 can generally determine a status 707S of each identifier generated. A status 707S can be merge, create and/or invalid. This determination can be made, by the comparing component 618, at least partially based upon use of, and indeed comparison to, library data 636 of the library datastore 635 that is desired to be updated.

For example, in one or more cases, the comparing component 618 can compare a first identifier 644 (such as the primary identifier 644, and/or an identifier based on highest ranking being first analyzed) and a library identifier 637 for the same chemical compound 631 to one another.

That is, the comparing component 618 can determine whether the identifier 644 comprises an annotation type 634A different from an annotation type 634A the library identifier 637. If the identifiers 644 and 637 do not have the same annotation type 634A, it can be determined whether any library identifier 637 for the chemical compound 631 at the library datastore 635 has a same annotation type 634A as the identifier 644, such as by searching the library datastore 635 and/or a metadata listing thereof. Where no match is found, the identifier 644 can be marked, by the comparing component 618, with a status of merge.

Additionally, and/or alternatively, the comparing component 618 can determine whether content 634C of the identifier 644 and a library identifier 637 for the same chemical compound 631, such as a highest ranked library identifier 637 for the same chemical compound 631, are consistent with one another, using a process as described above as performed by the prioritizing component 212, but instead now performed by the comparing component 618 and/or by the prioritizing component 212 aiding the comparing component 618. Where the identifiers 644, 637 are consistent, a status of merge can be employed and/or maintained for the identifier 644. Where the identifiers 644, 637 are inconsistent, a decision can be made by the comparing component 618, such as based on rules-based criteria submitted and/or specified by a user entity. For example, in one case, a highest ranking identifier 644, 637 can be employed and the other can be not employed. That is, a lower ranking identifier 644 can be not employed and marked with an invalid status 707S, or a lower ranking identifier 637 can be deleted or marked with an invalid status 707S with the higher ranking identifier 644 marked with or maintained with a status 707S of merge.

Additionally, and/or alternatively, the comparing component 618 can determine whether the identifier 644 outranks the library identifier 637 according to the rankings 642 thereof and/or according to the annotation ranking schema 640. The highest ranking 642 can be employed to resolve any consistency conflict, as noted above.

As a brief summary, the comparing component 618 can execute a first comparison comprising a first sub-comparison of forms of the annotation type 634A and the second annotation type 634A, a second sub-comparison of rankings (e.g., annotation rankings 642) of the annotation type 634A and the second annotation type 634A as compared to the annotation ranking schema 640, or both. That is, the comparing component 618 can determine whether to update the library datastore 635 with the identifier 644 based on a first determination resolving a first comparison of the annotation type 634A corresponding to the identifier 644 and a second annotation type 634A corresponding to the library identifier 637. Likewise, the comparing component 618 can determine whether to update the library datastore 635 with the identifier 644 based also on a second determination resolving a second comparison of consistency of a first content (e.g., identifier content 646) of the identifier 644 and a second content (e.g., library identifier content 638) of the library identifier 637.

Additionally, and/or alternatively, where no library identifiers 637 are discovered, by the comparing component 618 accessing the library database 635, for the chemical compound 631, the identifier 644 can be marked with a status 707S of create.

It is noted that after one or more comparisons, as noted above, have been performed, and/or at least partially in parallel with the one or more comparisons noted above, for a primary identifier 644, such one or more comparisons can also be performed for other secondary identifiers 644 that also have been generated for a same compound.

It is noted that any two or more of the above different comparisons can be performed, such as by the comparing component 618, at least partially in parallel with one another. Relative thereto, conflicts can be resolved in any suitable and/or specified order.

It is noted that in one or more cases, two or more comparisons can be performed at least partially in parallel with one another, where such two or more comparisons can be for the same identifier 644 as compared to different library identifiers 637 and/or different identifiers 644 compared to same or different library identifiers 637.

Based on the assignment and/or generation of statuses 707S, an optional sorting of the identifiers 644 by statuses 707S, such as within a category of a same chemical compound 631, can be performed at step 708 by the comparing component 618. Additionally, and/or alternatively, an option filtering out of identifiers 644 having a status 707S of invalid can be performed at step 708 by the comparing component 618.

At step 710, based a comparison having a status 707S of merge or update, and/or based on a determination by the comparing component 618 to perform an update 642, the updating component 620 can perform such update 642 of the library datastore 635 with the chemical compound data 631, and more particularly with the one or more identifiers 644 having been compared.

Turning now to the executing component 622, this component can generally generate a response 692 to a query 690 or to an inquiry comprised by a chemical compound input 630, such as where the query 690/input 630 includes an inquiry regarding a determination relative to a chemical compound 631. Such inquiry can comprise, for example, determining a classification, relationship, chemical family, closest spectra, identification of, similarity, difference etc., without being limited thereto, of the chemical compound 631 relative to the library data 636. For example, the executing component 622 can identify a classification for the chemical compound 631 based on one or more identifiers 644 having been added to the library datastore 635 as library identifiers 637. For example, that executing component 622 can return (e.g., generate a query response 692), for the query 690/input 630, plural identifiers 644 having been generated for the same chemical compound 631 based on the annotation ranking schema 640. That is, such plural identifiers 644 can be consistent with one another and have different annotation types 634A, yet be returned and analyzed for a same query 690/input 630.

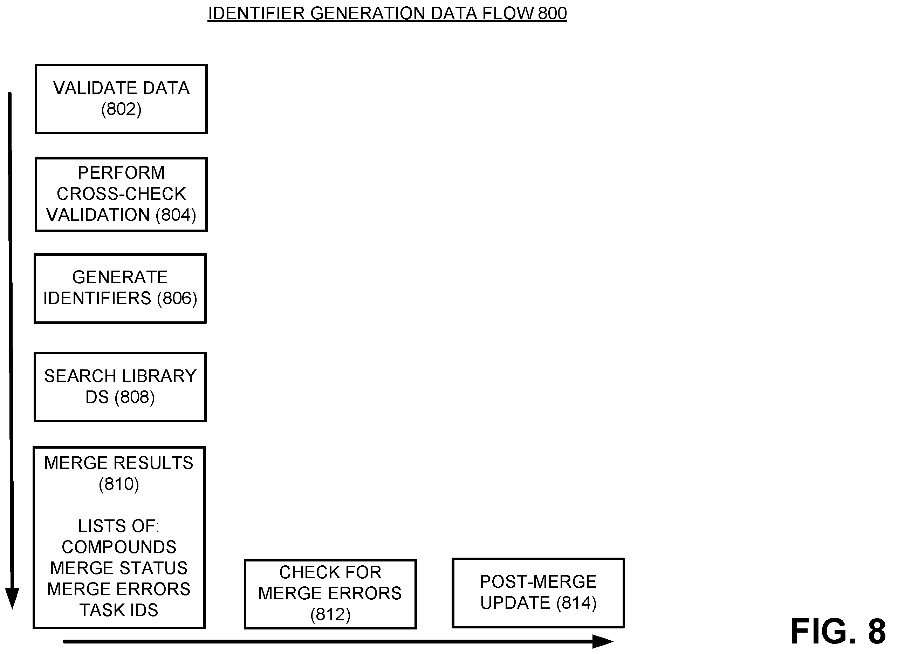

Turning next to FIG. 8, the identifier generation data flow 800 will be described as a first summary of the description of the one or more embodiments provided above. For example, a first step 608 can comprise validation of input chemical compound data 632 by the identifying component 610. The identifying component 610 further can perform one or more cross-check validations of varying aspects 633 of the chemical compound data 632 relative to one another at step 804. Further, one or more rankings 642 can be generated by the prioritizing component 612 based on the annotation ranking schema 640. At step 806, the generating component 616 can generate one or more identifiers 644. At step 808, the comparing component 618 can perform one or more of the varying comparisons described above using the library datastore 635 (e.g., library ds). At step 810, the updating component 620 can update the library datastore 635 based on the compounds 631, varying statuses 707S and can determine one or more merge errors and/or task IDs for logging one or more actions having been completed.

For example, at sub-step 812, the updating component 620 can check for one or more errors that may have occurred due to the update 642. Such errors can comprise loss of pre-existing identifiers 637 or library data 636 or unreadable and/or unreturnable data and/or metadata.

At sub-step 814, any one or more errors can be resolved using a post-merge update 642.

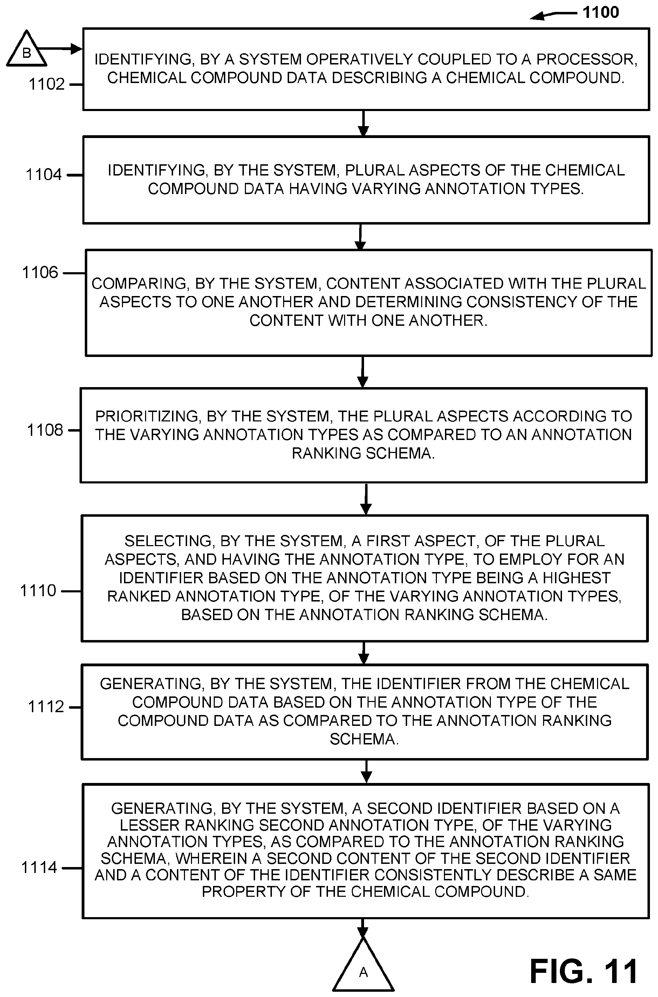

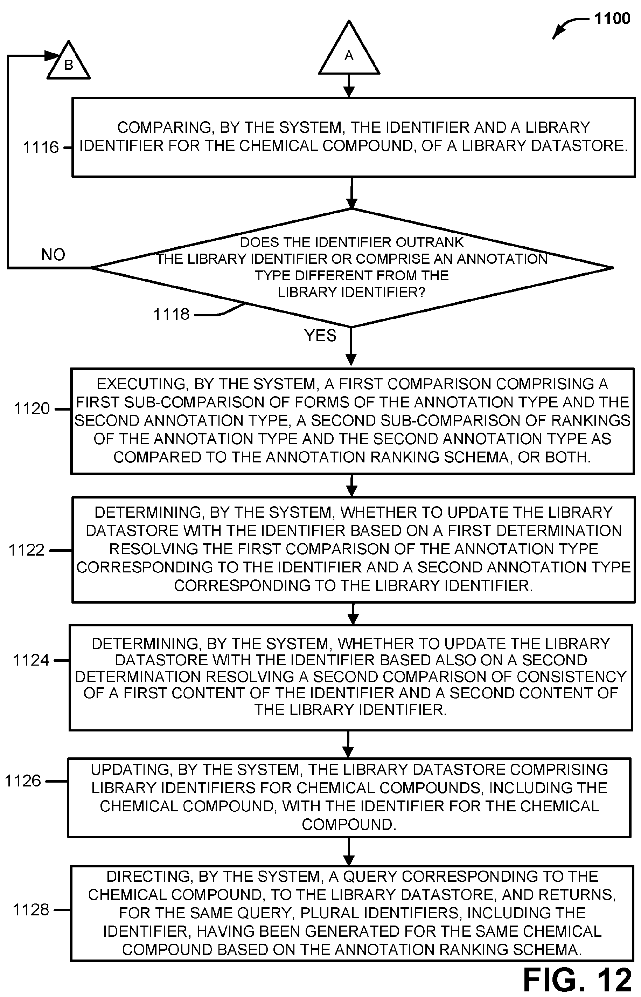

As a summary of the above-described components and/or functions thereof, referring next to FIGS. 11 and 12, illustrated is a flow diagram of an example, non-limiting method 1100 that can facilitate a process for identifier generation and subsequent updating of library content based on the identifier, in accordance with one or more embodiments described herein, such as the non-limiting system 600 of FIG. 6. While the non-limiting method 1100 is described relative to the non-limiting system 600 of FIG. 6, the non-limiting method 1100 can be applicable also to other systems described herein, such as the non-limiting system 500 of FIG. 5. Repetitive description of like elements and/or processes employed in respective embodiments is omitted for sake of brevity.

At 1102, the non-limiting method 1100 can comprise identifying, by a system (e.g., identifying component 610) chemical compound data (e.g., chemical compound data 632) describing a chemical compound (e.g., chemical compound 631).

At 1104, the non-limiting method 1100 can comprise identifying, by the system (e.g., identifying component 610), plural aspects (e.g., aspects 633) of the chemical compound data having varying annotation types (e.g., annotation types 634A).

At 1106, the non-limiting method 1100 can comprise comparing, by the system (e.g., prioritizing component 612), content (e.g., content 634C) associated with the plural aspects to one another and determining consistency of the content with one another.

At 1108, the non-limiting method 1100 can comprise prioritizing, by the system (e.g., prioritizing component 612), the plural aspects according to the varying annotation types as compared to an annotation ranking schema (e.g., annotation ranking schema 640).

At 1110, the non-limiting method 1100 can comprise selecting, by the system (e.g., selecting component 614) a first aspect, of the plural aspects, and having the annotation type, to employ for an identifier (e.g., identifier 644) based on the annotation type being a highest ranked annotation type, of the varying annotation types, based on the annotation ranking schema.

At 1112, the non-limiting method 1100 can comprise generating, by the system (e.g., generating component 616), the identifier from the chemical compound data based on the annotation type of the compound data as compared to the annotation ranking schema.

At 1114, the non-limiting method 1100 can comprise generating, by the system (e.g., generating component 616), a second identifier (e.g., identifiers 644) based on a lesser ranking second annotation type, of the varying annotation types, as compared to the annotation ranking schema, wherein a second content (e.g., identifier content 646) of the second identifier and a content (e.g., identifier content 646) of the identifier consistently describe a same property of the chemical compound.

At 1116, the non-limiting method 1100 can comprise comparing, by the system (e.g., comparing component 618), the identifier and a library identifier (e.g., library identifier 637) for the chemical compound, of a library datastore (e.g., library datastore 635).

At 1118, the non-limiting method 1100 can comprise determining, by the system (e.g., comparing component 618), whether the identifier outranks the library identifier or comprises an annotation type different from the library identifier. If not, the non-limiting method 1100 can proceed back to a next identifier (e.g., second identifier) having been generated, and if fully examined (e.g., a full set of identifiers generated), can proceed back to step 1102 for identification of additional chemical compound data. If yes, the non-limiting method 1100 can proceed to step 1120.

At 1120, the non-limiting method 1100 can comprise executing, by the system (e.g., updating component 620), a first comparison comprising a first sub-comparison of forms of the annotation type and the second annotation type, a second sub-comparison of rankings (e.g., annotation rankings 642) of the annotation type and the second annotation type as compared to the annotation ranking schema, or both.

At 1122, the non-limiting method 1100 can comprise determining, by the system (e.g., updating component 620), whether to update the library datastore with the identifier based on a first determination resolving a first comparison of the annotation type corresponding to the identifier and a second annotation type corresponding to the library identifier.

At 1124, the non-limiting method 1100 can comprise determining, by the system (e.g., updating component 620), whether to update the library datastore with the identifier based also on a second determination resolving a second comparison of consistency of a first content (e.g., identifier content 646) of the identifier and a second content (e.g., library identifier content 638) of the library identifier.

At 1126, the non-limiting method 1100 can comprise updating, by the system (e.g., updating component 620), the library datastore comprising library identifiers for chemical compounds, including the chemical compound, with the identifier (e.g., an update 642) for the chemical compound.

At 1128, the non-limiting method 1100 can comprise directing, by the system (e.g., executing component 622), a query (e.g., query 690) corresponding to the chemical compound, to the library datastore, and returns, for the same query, plural identifiers (e.g., query response 692), including the identifier, having been generated for the same chemical compound based on the annotation ranking schema.

### Additional Summary

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. In addition, the computer-implemented and non-computer-implemented methodologies could alternatively be represented as a series of interrelated states via a state diagram or events. Additionally, the computer-implemented methodologies described hereinafter and throughout this specification are capable of being stored on an article of manufacture for transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In summary, one or more systems, computer program products and/or computer-implemented methods provided herein relate to a process for generation of annotation-accessible library spectral content (e.g., library data 636). A system (e.g., identifier generation system 502, 602) can comprise a memory (e.g., memory 504, 604) that stores, and a processor (e.g., processor 506, 606) that executes, computer executable components. The computer executable components can comprise an identifying component (e.g., identifying component 510, 610) that identifies chemical compound data (e.g., chemical compound data 532, 632) describing a chemical compound (e.g., chemical compound 531, 631), and a generating component (e.g., generating component 516, 616) that generates an identifier (e.g., identifier 544, 644) from the chemical compound data based on an annotation type (e.g., annotation type 534, 634A) of the compound data as compared to an annotation ranking schema (e.g., annotation ranking schema 540, 640).

The one or more embodiments described herein can employ a novel system that provides for generation and use of varying annotation types across different identifiers for same compounds and/or different compounds of a library datastore without consistency issues between the identifiers having been generated by the one or more embodiments described herein. In this way, queries to such library can be returned relative to one or relative to plural annotation types, regardless of an annotation type associated with a query and/or an annotation type associated with library data of the library. Thus, cross-referencing of similarities, differences and/or relationships between compounds (including spectra of the compounds) of such library can be facilitated and executed with ease and efficiency.

Indeed, in view of the one or more embodiments described herein, a practical application of the one or more systems, computer-implemented methods and/or computer program products described herein can be ability to provide for consistency of content of identifiers generated for same compounds but based on different annotation types. That is, the one or more identifiers generated for a same compound can be consistent such as to not be in conflict with one another (e.g., describing the compound as having conflicting properties, for example). This can be facilitated by the use of the annotation ranking schema and various cross-checks and/or comparisons performed by the one or more embodiments described herein. That is, as compared to existing frameworks that cannot provide this ability, and which do not comprise consideration of other annotation types when generating an identifier, the one or more embodiments described herein can provide a new library search ability that was previously unavailable.

These are useful and practical applications of computers, thus providing enhanced (e.g., improved and/or optimized) compound analysis and/or spectra analysis output. Overall, such computerized tools can constitute a concrete and tangible technical improvement in the fields of material analysis, and more particularly in material analysis using a network of library spectral content for purposes of cross-referencing similarities, differences and/or relationships between different library spectral content.

Furthermore, one or more embodiments described herein can be employed in a real-world system based on the disclosed teachings. For example, the one or more embodiments described herein can provide generation of identifiers for compound data based on varying annotation types comprising varying text, code and/or metadata. Indeed, a benefit of the one or more embodiments described herein is an ability to differentiate between such varying annotation types for generating the one or more identifiers. Based thereon, the one or more embodiments described herein can be employed to generate the identifiers such that a library updated with the identifiers can be searched in an annotation type-agnostic manner. That is, information can be returned in response to a query that is based on different annotation types (e.g., plural annotation types) for a same compound. Accordingly, a search employing one annotation type can return data from another annotation type that has already been cross-checked for consistency and/or labeled for ranking based on the annotation ranking schema. These can be useful processes for varying industries employing material analysis, product manufacturing, quality control and/or the like. The embodiments disclosed herein thus can provide improvements to scientific instrument technology (e.g., improvements in the computer technology supporting such scientific instruments, among other improvements).

Further in one or more cases, the embodiments described herein can be self-improving. Indeed, as identifiers are generated for compound data based on varying annotation types and a corresponding annotation ranking schema, comparisons for determining whether or not to update a library can become more efficient and accurate over time. That is, as more library content is added by the embodiments described herein, a larger body of accurate comparative data is generated for use in searches, queries, and/other comparisons performed relative to the various cross-checks employed by the one or more embodiments described herein to generate the identifiers for the spectral content in the first instance.

In addition, in one or more embodiments, the annotation ranking schema employed to prioritize the different annotation types for a same compound or spectrum for a compound, can be customized at any suitable time to adjust ranking data employed to provide rankings for the varying annotation types of input compound data (e.g., input to a non-limiting system described herein).

The one or more embodiments described herein can be implemented within, in connection with and/or coupled to a scientific imaging device.

The one or more embodiments described herein can be applied on a plug-and-play basis to various architectures of existing spectral library and/or library datastores of spectral data. That is, the one or more embodiments described herein can generate identifiers for compounds (including for spectra corresponding to compounds) regardless of data structure of a spectral library and/or library datastore.

Moreover, the one or more embodiments described herein can achieve a level of scale of operation. For example, two or more aspects of a compound data for a same compound, or even two or more sets of compound data for different compounds, can be analyzed and identifiers generated therefor, at least partially in parallel with one another. In one or more cases, a library, or even two or more libraries, can thus be updated at least partially in parallel with one another and/or in parallel with one or more identifier generations relative to one or more compound data sets.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

One or more embodiments described herein can be, in one or more embodiments, inherently and/or inextricably tied to computer technology and cannot be implemented outside of a computing environment. For example, one or more processes performed by one or more embodiments described herein can more efficiently, and even more feasibly, provide program and/or program instruction execution, such as relative to chemical compound analysis using annotation types, such as computerized and/or computer code annotation types, as compared to existing systems and/or techniques for generation of library spectral content. Systems, computer-implemented methods and/or computer program products providing performance of these processes are of great utility in the fields of material analysis, such for determining one or more chemical correspondences (e.g., chemical properties, relationships and/or classification) for one or more compound queries and cannot be equally practicably implemented in a sensible way outside of a computing environment.

One or more embodiments described herein can employ hardware and/or software to solve problems that are highly technical, that are not abstract, and that cannot be performed as a set of mental acts by a human. For example, a human, or even thousands of humans, cannot efficiently, accurately and/or effectively analyze computer data/metadata defining a plurality of compounds, or defining spectra for a plurality of compounds, and/or generate computer-usable metadata identifiers for a computer-based search of library data stored at a memory device, as the one or more embodiments described herein can provide this process. Moreover, neither can the human mind nor a human with pen and paper conduct one or more of these processes, as conducted by one or more embodiments described herein.

In one or more embodiments, one or more of the processes described herein can be performed by one or more specialized computers (e.g., a specialized processing unit, a specialized classical computer, and/or another type of specialized computer) to execute defined tasks related to the one or more technologies describe above. One or more embodiments described herein and/or components thereof can be employed to solve new problems that arise through advancements in technologies mentioned above, employment of cloud computing systems, computer architecture and/or another technology.

One or more embodiments described herein can be fully operational towards performing one or more other functions (e.g., fully powered on, fully executed and/or another function) while also performing one or more of the one or more operations described herein.

To provide additional summary, a listing of embodiments and features thereof is next provided.

A system, comprising: a memory that stores computer executable components; and a processor that executes the computer executable components stored in the memory, wherein the computer executable components comprise: an identifying component that identifies chemical compound data describing a chemical compound; and a generating component that generates an identifier from the chemical compound data based on an annotation type of the compound data as compared to an annotation ranking schema.

The system of the preceding paragraph, wherein the identifying component identifies plural aspects of the chemical compound data having varying annotation types, including the annotation type, and wherein the computer executable components further comprise a selecting component that selects a first aspect, of the plural aspects, and having the annotation type, to employ for the identifier based on the annotation type being a highest ranked annotation type, of the varying annotation types, based on the annotation ranking schema.

The system of any preceding paragraph, wherein the computer executable components further comprise: a prioritizing component that prioritizes the plural aspects according to the varying annotation types as compared to the annotation ranking schema.

The system of any preceding paragraph, wherein the generating component further generates a second identifier based on a lesser ranking second annotation type, of the varying annotation types, as compared to the annotation ranking schema, and wherein a second content of the second identifier and a content of the identifier consistently describe a same property of the chemical compound.

The system of any preceding paragraph, wherein the computer executable components further comprise: an updating component that updates a library datastore comprising library identifiers for chemical compounds, including the chemical compound, with the identifier for the chemical compound; and an executing component that directs a query corresponding to the chemical compound, to the library datastore, and returns, for the same query, plural identifiers, including the identifier, having been generated for the same chemical compound based on the annotation ranking schema.

The system of any preceding paragraph, wherein the computer executable components further comprise: a comparing component that compares the identifier and a library identifier for the chemical compound, of a library datastore; and an updating component that determines whether to update the library datastore with the identifier based on a first determination resolving a first comparison of the annotation type corresponding to the identifier and a second annotation type corresponding to the library identifier.

The system of any preceding paragraph, wherein the first comparison comprises a first sub-comparison of forms of the annotation type and the second annotation type, a second sub-comparison of rankings of the annotation type and the second annotation type as compared to the annotation ranking schema, or both.

The system of any preceding paragraph, wherein the updating component further determines whether to update the library datastore with the identifier based also on a second determination resolving a second comparison of consistency of a first content of the identifier and a second content of the library identifier.

A computer-implemented method, comprising: identifying, by a system operatively coupled to a processor, chemical compound data describing a chemical compound; and generating, by the system, an identifier from the chemical compound data based on an annotation type of the compound data as compared to an annotation ranking schema.

The computer-implemented method of the preceding paragraph, further comprising: identifying, by the system, plural aspects of the chemical compound data having varying annotation types, including the annotation type; and selecting, by the system, a first aspect, of the plural aspects, and having the annotation type, to employ for the identifier based on the annotation type being a highest ranked annotation type, of the varying annotation types, based on the annotation ranking schema.

The computer-implemented method of any preceding paragraph, further comprising: prioritizing, by the system, the plural aspects according to the varying annotation types as compared to the annotation ranking schema.

The computer-implemented method of any preceding paragraph, further comprising: generating, by the system, a second identifier based on a lesser ranking second annotation type, of the varying annotation types, as compared to the annotation ranking schema, wherein a second content of the second identifier and a content of the identifier consistently describe a same property of the chemical compound.

The computer-implemented method of any preceding paragraph, further comprising: updating, by the system, a library datastore comprising library identifiers for chemical compounds, including the chemical compound, with the identifier for the chemical compound; and directing, by the system, a query corresponding to the chemical compound, to the library datastore, and returns, for the same query, plural identifiers, including the identifier, having been generated for the same chemical compound based on the annotation ranking schema.

The computer-implemented method of any preceding paragraph, further comprising: comparing, by the system, the identifier and a library identifier for the chemical compound, of a library datastore; and determining, by the system, whether to update the library datastore with the identifier based on a first determination resolving a first comparison of the annotation type corresponding to the identifier and a second annotation type corresponding to the library identifier, wherein the first comparison comprises a first sub-comparison of forms of the annotation type and the second annotation type, a second sub-comparison of rankings of the annotation type and the second annotation type as compared to the annotation ranking schema, or both.

The computer-implemented method of any preceding paragraph, further comprising: determining, by the system, whether to update the library datastore with the identifier based also on a second determination resolving a second comparison of consistency of a first content of the identifier and a second content of the library identifier.

A computer program product facilitating a process for generating chemical compound identifiers based on varying annotation types, the computer program product comprising a computer readable storage medium having program instructions embodied therewith, and the program instructions executable by a processor to cause the processor to: identify, by the processor, chemical compound data describing a chemical compound; and generate, by the processor, an identifier from the chemical compound data based on an annotation type of the compound data as compared to an annotation ranking schema.

The computer program product of the preceding paragraph, wherein the program instructions are further executable by the processor to cause the processor to: identify, by the processor, plural aspects of the chemical compound data having varying annotation types, including the annotation type; and select, by the processor, a first aspect, of the plural aspects, and having the annotation type, to employ for the identifier based on the annotation type being a highest ranked annotation type, of the varying annotation types, based on the annotation ranking schema.

The computer program product of any preceding paragraph, wherein the program instructions are further executable by the processor to cause the processor to: prioritize, by the processor, the plural aspects according to the varying annotation types as compared to the annotation ranking schema.

The computer program product of any preceding paragraph, wherein the program instructions are further executable by the processor to cause the processor to: generate, by the processor, a second identifier based on a lesser ranking second annotation type, of the varying annotation types, as compared to the annotation ranking schema, wherein a second content of the second identifier and a content of the identifier consistently describe a same property of the chemical compound.

The computer program product of any preceding paragraph, wherein the program instructions are further executable by the processor to cause the processor to: update, by the processor, a library datastore comprising library identifiers for chemical compounds, including the chemical compound, with the identifier for the chemical compound; and direct, by the processor, a query corresponding to the chemical compound, to the library datastore, and returns, for the same query, plural identifiers, including the identifier, having been generated for the same chemical compound based on the annotation ranking schema.

### Scientific Instrument System Description

Turning next to FIG. 13, a detailed description is provided of additional context for the one or more embodiments described herein at FIGS. 1-12. One or more computing devices implementing any of the scientific instrument modules or methods disclosed herein can be part of a scientific instrument system. FIG. 13 illustrates a block diagram of an example scientific instrument system 1300 in which one or more of the scientific instrument methods or other methods disclosed herein can be performed, in accordance with various embodiments described herein. The scientific instrument modules and methods disclosed herein (e.g., the scientific instrument module 100 of FIG. 1 and the method 200 of FIG. 2) can be implemented by one or more of the scientific instrument 1310, the user local computing device 1320, the service local computing device 1330, and/or the remote computing device 1340 of the scientific instrument system 1300.

Any of the scientific instrument 1310, the user local computing device 1320, the service local computing device 1330, and/or the remote computing device 1340 can include any of the embodiments of the computing device 400 discussed herein with reference to FIG. 4, and any of the scientific instrument 1310, the user local computing device 1320, the service local computing device 1330, and/or the remote computing device 1340 can take the form of any appropriate one or more of the embodiments of the computing device 400 discussed herein with reference to FIG. 4.

One or more of the scientific instrument 1310, the user local computing device 1320, the service local computing device 1330, and/or the remote computing device 1340 can include a processing device 1302, a storage device 1304, and/or an interface device 1306. The processing device 1302 can take any suitable form, including the form of any of the processors 402 discussed herein with reference to FIG. 4. The processing devices 1302 included in different ones of the scientific instrument 1310, the user local computing device 1320, the service local computing device 1330, and/or the remote computing device 1340 can take the same form or different forms. The storage device 1304 can take any suitable form, including the form of any of the storage devices 404 discussed herein with reference to FIG. 4. The storage devices 1304 included in different ones of the scientific instrument 1310, the user local computing device 1320, the service local computing device 1330, and/or the remote computing device 1340 can take the same form or different forms. The interface device 1306 can take any suitable form, including the form of any of the interface devices 406 discussed herein with reference to FIG. 4. The interface devices 1306 included in different ones of the scientific instrument 1310, the user local computing device 1320, the service local computing device 1330, and/or the remote computing device 1340 can take the same form or different forms.

The scientific instrument 1310, the user local computing device 1320, the service local computing device 1330, and/or the remote computing device 1340 can be in communication with other elements of the scientific instrument system 1300 via communication pathways 1308. The communication pathways 1308 can communicatively couple the interface devices 1306 of different ones of the elements of the scientific instrument system 1300, as shown, and can be wired or wireless communication pathways (e.g., in accordance with any of the communication techniques discussed herein with reference to the interface devices 406 of the computing device 400 of FIG. 4). The particular scientific instrument system 1300 depicted in FIG. 13 includes communication pathways between each pair of the scientific instrument 1310, the user local computing device 1320, the service local computing device 1330, and the remote computing device 1340, but this "fully connected" implementation is simply illustrative, and in various embodiments, various ones of the communication pathways 1308 can be omitted. For example, in one or more embodiments, a service local computing device 1330 can omit a direct communication pathway 1308 between its interface device 1306 and the interface device 1306 of the scientific instrument 1310, but can instead communicate with the scientific instrument 1310 via the communication pathway 1308 between the service local computing device 1330 and the user local computing device 1320 and/or the communication pathway 1308 between the user local computing device 1320 and the scientific instrument 1310.

The scientific instrument 1310 can include any appropriate scientific instrument, such as a separation or MS instrument, or other instrument facilitating material analysis.

The user local computing device 1320 can be a computing device (e.g., in accordance with any of the embodiments of the computing device 400 discussed herein) that is local to a user of the scientific instrument 1310. In one or more embodiments, the user local computing device 1320 can also be local to the scientific instrument 1310, but this need not be the case; for example, a user local computing device 1320 that is associated with a home, office or other building associated with a user entity can be remote from, but in communication with, the scientific instrument 1310 so that the user entity can use the user local computing device 1320 to control and/or access data from the scientific instrument 1310. In one or more embodiments, the user local computing device 1320 can be a laptop, smartphone, or tablet device. In one or more embodiments the user local computing device 1320 can be a portable computing device. In one or more embodiments, the user local computing device 1320 can deployed in the field.

The service local computing device 1330 can be a computing device (e.g., in accordance with any of the embodiments of the computing device 400 discussed herein) that is local to an entity that services the scientific instrument 1310. For example, the service local computing device 1330 can be local to a manufacturer of the scientific instrument 1310 or to a third-party service company. In one or more embodiments, the service local computing device 1330 can communicate with the scientific instrument 1310, the user local computing device 1320, and/or the remote computing device 1340 (e.g., via a direct communication pathway 1308 or via multiple "indirect" communication pathways 1308, as discussed above) to receive data regarding the operation of the scientific instrument 1310, the user local computing device 1320, and/or the remote computing device 1340 (e.g., the results of self-tests of the scientific instrument 1310, calibration coefficients used by the scientific instrument 1310, the measurements of sensors associated with the scientific instrument 1310, etc.). In one or more embodiments, the service local computing device 1330 can communicate with the scientific instrument 1310, the user local computing device 1320, and/or the remote computing device 1340 (e.g., via a direct communication pathway 1308 or via multiple "indirect" communication pathways 1308, as discussed above) to transmit data to the scientific instrument 1310, the user local computing device 1320, and/or the remote computing device 1340 (e.g., to update programmed instructions, such as firmware, in the scientific instrument 1310, to initiate the performance of test or calibration sequences in the scientific instrument 1310, to update programmed instructions, such as software, in the user local computing device 1320 or the remote computing device 1340, etc.). A user entity of the scientific instrument 1310 can utilize the scientific instrument 1310 or the user local computing device 1320 to communicate with the service local computing device 1330 to report a problem with the scientific instrument 1310 or the user local computing device 1320, to request a visit from a technician to improve the operation of the scientific instrument 1310, to order consumables or replacement parts associated with the scientific instrument 1310, or for other purposes.

The remote computing device 1340 can be a computing device (e.g., in accordance with any of the embodiments of the computing device 400 discussed herein) that is remote from the scientific instrument 1310 and/or from the user local computing device 1320. In one or more embodiments, the remote computing device 1340 can be included in a datacenter or other large-scale server environment. In one or more embodiments, the remote computing device 1340 can include network-attached storage (e.g., as part of the storage device 1304). The remote computing device 1340 can store data generated by the scientific instrument 1310, perform analyses of the data generated by the scientific instrument 1310 (e.g., in accordance with programmed instructions), facilitate communication between the user local computing device 1320 and the scientific instrument 1310, and/or facilitate communication between the service local computing device 1330 and the scientific instrument 1310.

In one or more embodiments, one or more of the elements of the scientific instrument system 1300 illustrated in FIG. 13 can be omitted. Further, in one or more embodiments, multiple ones of various ones of the elements of the scientific instrument system 1300 of FIG. 13 can be present. For example, a scientific instrument system 1300 can include multiple user local computing devices 1320 (e.g., different user local computing devices 1320 associated with different user entities or in different locations). In another example, a scientific instrument system 1300 can include multiple scientific instruments 1310, all in communication with service local computing device 1330 and/or a remote computing device 1340; in such an embodiment, the service local computing device 1330 can monitor these multiple scientific instruments 1310, and the service local computing device 1330 can cause updates or other information can be "broadcast" to multiple scientific instruments 1310 at the same time. Different ones of the scientific instruments 1310 in a scientific instrument system 1300 can be located close to one another (e.g., in the same room) or farther from one another (e.g., on different floors of a building, in different buildings, in different cities, etc.). In one or more embodiments, a scientific instrument 1310 can be connected to an Internet-of-Things (IoT) stack that allows for command and control of the scientific instrument 1310 through a web-based application, a virtual or augmented reality application, a mobile application, and/or a desktop application. Any of these applications can be accessed by a user entity operating the user local computing device 1320 in communication with the scientific instrument 1310 by the intervening remote computing device 1340. In one or more embodiments, a scientific instrument 1310 can be sold by the manufacturer along with one or more associated user local computing devices 1320 as part of a local scientific instrument computing unit 1312.

In one or more embodiments, different ones of the scientific instruments 1310 included in a scientific instrument system 1300 can be different types of scientific instruments 1310; for example, one scientific instrument 1310 can be an EDS device, while another scientific instrument 1310 can be an analysis device that analyzes results of an EDS device. In some such embodiments, the remote computing device 1340 and/or the user local computing device 1320 can combine data from different types of scientific instruments 1310 included in a scientific instrument system 1300.

### Example Operating Environment

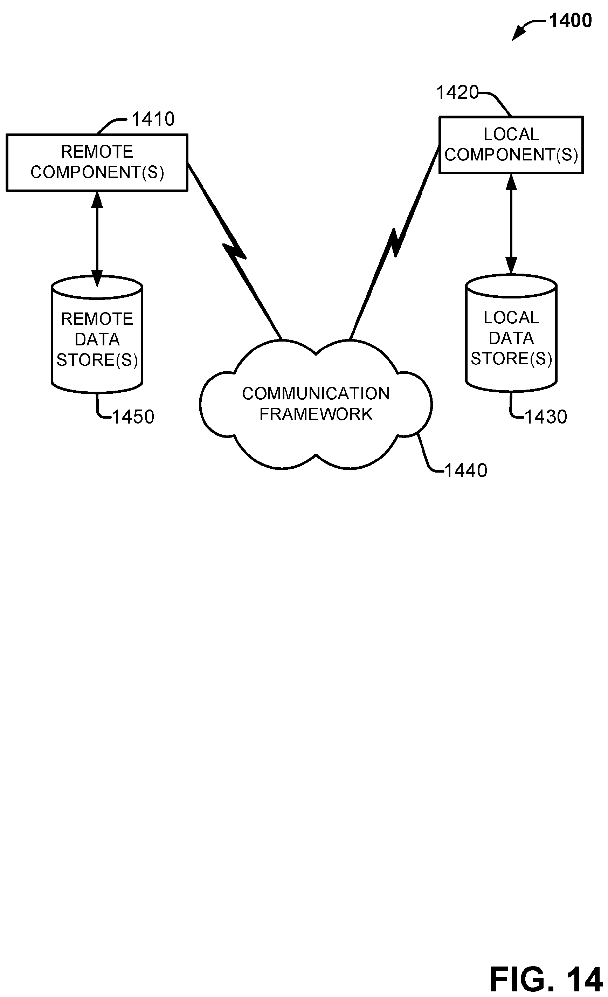

FIG. 14 is a schematic block diagram of an operating environment 1400 with which the described subject matter can interact. The operating environment 1400 comprises one or more remote component(s) 1410. The remote component(s) 1410 can be hardware and/or software (e.g., threads, processes, computing devices). In one or more embodiments, remote component(s) 1410 can be a distributed computer system, connected to a local automatic scaling component and/or programs that use the resources of a distributed computer system, via communication framework 1440. Communication framework 1440 can comprise wired network devices, wireless network devices, mobile devices, wearable devices, radio access network devices, gateway devices, femtocell devices, servers, etc.

The operating environment 1400 also comprises one or more local component(s) 1420. The local component(s) 1420 can be hardware and/or software (e.g., threads, processes, computing devices). In one or more embodiments, local component(s) 1420 can comprise an automatic scaling component and/or programs that communicate/use the remote resources 1410 and 1420, etc., connected to a remotely located distributed computing system via communication framework 1440.

One possible communication between a remote component(s) 1410 and a local component(s) 1420 can be in the form of a data packet adapted to be transmitted between two or more computer processes. Another possible communication between a remote component(s) 1410 and a local component(s) 1420 can be in the form of circuit-switched data adapted to be transmitted between two or more computer processes in radio time slots. The operating environment 1400 comprises a communication framework 1440 that can be employed to facilitate communications between the remote component(s) 1410 and the local component(s) 1420, and can comprise an air interface, e.g., interface of a UMTS network, via an LTE network, etc. Remote component(s) 1410 can be operably connected to one or more remote datastore(s) 1450, such as a hard drive, solid state drive, subscriber identity module (SIM) card, electronic SIM (eSIM), device memory, etc., that can be employed to store information on the remote component(s) 1410 side of communication framework 1440. Similarly, local component(s) 1420 can be operably connected to one or more local datastore(s) 1430, that can be employed to store information on the local component(s) 1420 side of communication framework 1440.

### Example Computing Environment

In order to provide additional context for various embodiments described herein, FIG. 15 and the following discussion are intended to provide a brief, general description of a suitable computing environment 1500 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform tasks or implement abstract data types. Moreover, the methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

Referring still to FIG. 15, the example computing environment 1500 which can implement one or more embodiments described herein includes a computer 1502, the computer 1502 including a processing unit 1504, a system memory 1506 and a system bus 1508. The system bus 1508 couples system components including, but not limited to, the system memory 1506 to the processing unit 1504. The processing unit 1504 can be any of various commercially available processors. Dual microprocessors and other multi processor architectures can also be employed as the processing unit 1504.

The system bus 1508 can be any of several types of bus structure that can further interconnect to a memory bus (with or without a memory controller), a peripheral bus, and a local bus using any of a variety of commercially available bus architectures. The system memory 1506 includes ROM 1510 and RAM 1512. A basic input/output system (BIOS) can be stored in a non-volatile memory such as ROM, erasable programmable read only memory (EPROM), EEPROM, which BIOS contains the basic routines that help to transfer information between elements within the computer 1502, such as during startup. The RAM 1512 can also include a high-speed RAM such as static RAM for caching data.

The computer 1502 further includes an internal hard disk drive (HDD) 1514 (e.g., EIDE, SATA), and can include one or more external storage devices 1516 (e.g., a magnetic floppy disk drive (FDD) 1516, a memory stick or flash drive reader, a memory card reader, etc.). While the internal HDD 1514 is illustrated as located within the computer 1502, the internal HDD 1514 can also be configured for external use in a suitable chassis (not shown). Additionally, while not shown in computing environment 1500, a solid-state drive (SSD) could be used in addition to, or in place of, an HDD 1514.

Other internal or external storage can include at least one other storage device 1520 with storage media 1522 (e.g., a solid-state storage device, a nonvolatile memory device, and/or an optical disk drive that can read or write from removable media such as a CD-ROM disc, a DVD, a BD, etc.). The external storage 1516 can be facilitated by a network virtual machine. The HDD 1514, external storage device 1516 and storage device (e.g., drive) 1520 can be connected to the system bus 1508 by an HDD interface 1524, an external storage interface 1526 and a drive interface 1528, respectively.

The drives and their associated computer-readable storage media provide nonvolatile storage of data, data structures, computer-executable instructions, and so forth. For the computer 1502, the drives and storage media accommodate the storage of any data in a suitable digital format. Although the description of computer-readable storage media above refers to respective types of storage devices, other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1512, including an operating system 1530, one or more application programs 1532, other program modules 1534 and program data 1536. All or portions of the operating system, applications, modules, and/or data can also be cached in the RAM 1512. The systems and methods described herein can be implemented utilizing various commercially available operating systems or combinations of operating systems.

Computer 1502 can optionally comprise emulation technologies. For example, a hypervisor (not shown) or other intermediary can emulate a hardware environment for operating system 1530, and the emulated hardware can optionally be different from the hardware illustrated in FIG. 15. In such an embodiment, operating system 1530 can comprise one virtual machine (VM) of multiple VMs hosted at computer 1502. Furthermore, operating system 1530 can provide runtime environments, such as the Java runtime environment or the .NET framework, for applications 1532. Runtime environments are consistent execution environments that allow applications 1532 to run on any operating system that includes the runtime environment. Similarly, operating system 1530 can support containers, and applications 1532 can be in the form of containers, which are lightweight, standalone, executable packages of software that include, e.g., code, runtime, system tools, system libraries and settings for an application.

Further, computer 1502 can be enabled with a security module, such as a trusted processing module (TPM). For instance, with a TPM, boot components hash next in time boot components, and wait for a match of results to secured values, before loading a next boot component. This process can take place at any layer in the code execution stack of computer 1502, e.g., applied at the application execution level or at the operating system (OS) kernel level, thereby enabling security at any level of code execution.

A user entity can enter commands and information into the computer 1502 through one or more wired/wireless input devices, e.g., a keyboard 1538, a touch screen 1540, and a pointing device, such as a mouse 1542. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera, a gesture sensor input device, a vision movement sensor input device, an emotion or facial detection device, a biometric input device, e.g., fingerprint or iris scanner, or the like. These and other input devices are often connected to the processing unit 1504 through an input device interface 1544 that can be coupled to the system bus 1508, but can be connected by other interfaces, such as a parallel port, an IEEE 1394 serial port, a game port, a USB port, an IR interface, a BLUETOOTH^{®} interface, etc.

A monitor 1546 or other type of display device can also be connected to the system bus 1508 via an interface, such as a video adapter 1548. In addition to the monitor 1546, a computer typically includes other peripheral output devices (not shown), such as speakers, printers, etc.

The computer 1502 can operate in a networked environment using logical connections via wired and/or wireless communications to one or more remote computers, such as a remote computer 1550. The remote computer 1550 can be a workstation, a server computer, a router, a personal computer, portable computer, microprocessor-based entertainment appliance, a peer device or other common network node, and typically includes many or all of the elements described relative to the computer 1502, although, for purposes of brevity, only a memory/storage device 1552 is illustrated. The logical connections depicted include wired/wireless connectivity to a local area network (LAN) 1554 and/or larger networks, e.g., a wide area network (WAN) 1556. Such LAN and WAN networking environments are commonplace in offices and companies, and facilitate enterprise-wide computer networks, such as intranets, all of which can connect to a global communications network, e.g., the Internet.

When used in a LAN networking environment, the computer 1502 can be connected to the local network 1554 through a wired and/or wireless communication network interface or adapter 1558. The adapter 1558 can facilitate wired or wireless communication to the LAN 1554, which can also include a wireless access point (AP) disposed thereon for communicating with the adapter 1558 in a wireless mode.

When used in a WAN networking environment, the computer 1502 can include a modem 1560 or can be connected to a communications server on the WAN 1556 via other means for establishing communications over the WAN 1556, such as by way of the Internet. The modem 1560, which can be internal or external and a wired or wireless device, can be connected to the system bus 1508 via the input device interface 1544. In a networked environment, program modules depicted relative to the computer 1502 or portions thereof, can be stored in the remote memory/storage device 1552. The network connections shown are example and other means of establishing a communications link between the computers can be used.

When used in either a LAN or WAN networking environment, the computer 1502 can access cloud storage systems or other network-based storage systems in addition to, or in place of, external storage devices 1516 as described above. Generally, a connection between the computer 1502 and a cloud storage system can be established over a LAN 1554 or WAN 1556 e.g., by the adapter 1558 or modem 1560, respectively. Upon connecting the computer 1502 to an associated cloud storage system, the external storage interface 1526 can, with the aid of the adapter 1558 and/or modem 1560, manage storage provided by the cloud storage system as it would other types of external storage. For instance, the external storage interface 1526 can be configured to provide access to cloud storage sources as if those sources were physically connected to the computer 1502.

The computer 1502 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a defined structure as with an existing network or simply an ad hoc communication between at least two devices.

### Additional Information

The embodiments described herein can be directed to one or more of a system, a method, an apparatus and/or a computer program product at any possible technical detail level of integration. The computer program product can include a computer readable storage medium (or media) having computer readable program instructions thereon for causing a processor to carry out aspects of the one or more embodiments described herein. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a superconducting storage device and/or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, is not to be construed as being transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein can be downloaded to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more embodiments, electronic circuitry including, for example, programmable logic circuitry, field-programmable gate arrays (FPGA) and/or programmable logic arrays (PLA) can execute the computer readable program instructions by utilizing state information of the computer readable program instructions to personalize the electronic circuitry, in order to perform aspects of the one or more embodiments described herein.

Aspects of the one or more embodiments described herein are described with reference to flowchart illustrations and/or block diagrams of methods, apparatus (systems), and computer program products according to one or more embodiments described herein. It will be understood that each block of the flowchart illustrations and/or block diagrams, and combinations of blocks in the flowchart illustrations and/or block diagrams, can be implemented by computer readable program instructions. These computer readable program instructions can be provided to a processor of a general-purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computer-implementable methods and/or computer program products according to one or more embodiments described herein. In this regard, each block in the flowchart or block diagrams can represent a module, segment and/or portion of instructions, which comprises one or more executable instructions for implementing the specified logical function. In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter has been described above in the general context of computer-executable instructions of a computer program product that runs on a computer and/or computers, those skilled in the art will recognize that the one or more embodiments herein also can be implemented at least partially in parallel with one or more other program modules. Generally, program modules include routines, programs, components and/or data structures that perform particular tasks and/or implement particular abstract data types. Moreover, the aforedescribed computer-implemented methods can be practiced with other computer system configurations, including single-processor and/or multiprocessor computer systems, mini-computing devices, mainframe computers, as well as computers, hand-held computing devices (e.g., PDA, phone), and/or microprocessor-based or programmable consumer and/or industrial electronics. The illustrated aspects can also be practiced in distributed computing environments in which tasks are performed by remote processing devices that are linked through a communications network. However, one or more, if not all aspects of the one or more embodiments described herein can be practiced on stand-alone computers. In a distributed computing environment, program modules can be located in both local and remote memory storage devices.

As used in this application, the terms "component," "system," "platform" and/or "interface" can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form. As used herein, the terms "example" and/or "exemplary" are utilized to mean serving as an example, instance, or illustration. For the avoidance of doubt, the subject matter described herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

As it is employed in the subject specification, the term "processor" can refer to substantially any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nano-scale architectures such as, but not limited to, molecular based transistors, switches and/or gates, in order to optimize space usage and/or to enhance performance of related equipment. A processor can be implemented as a combination of computing processing units.

Herein, terms such as "store," "storage," "datastore," data storage," "database," and substantially any other information storage component relevant to operation and functionality of a component are utilized to refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory and/or nonvolatile random-access memory (RAM) (e.g., ferroelectric RAM (FeRAM). Volatile memory can include RAM, which can act as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein are intended to include, without being limited to including, these and/or any other suitable types of memory.

What has been described above includes mere examples of systems and computer-implemented methods. It is, of course, not possible to describe every conceivable combination of components and/or computer-implemented methods for purposes of describing the one or more embodiments, but one of ordinary skill in the art can recognize that many further combinations and/or permutations of the one or more embodiments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the various embodiments can use the phrases "an embodiment," "various embodiments," "one or more embodiments" and/or "some embodiments," each of which can refer to one or more of the same or different embodiments.

The descriptions of the various embodiments have been presented for purposes of illustration but are not intended to be exhaustive or limited to the embodiments described herein. Many modifications and variations will be apparent to those of ordinary skill in the art without departing from the scope and spirit of the described embodiments. The terminology used herein was chosen to best explain the principles of the embodiments, the practical application and/or technical improvement over technologies found in the marketplace, and/or to enable others of ordinary skill in the art to understand the embodiments described herein.

## Claims

1. A computer-implemented method, comprising:
identifying, by a system operatively coupled to a processor, chemical compound data describing a chemical compound; and
generating, by the system, an identifier from the chemical compound data based on an annotation type of the compound data as compared to an annotation ranking schema.

2. The computer-implemented method of claim 1, further comprising:
identifying, by the system, plural aspects of the chemical compound data having varying annotation types, including the annotation type; and
selecting, by the system, a first aspect, of the plural aspects, and having the annotation type, to employ for the identifier based on the annotation type being a highest ranked annotation type, of the varying annotation types, based on the annotation ranking schema.

3. The computer-implemented method of claim 2, further comprising:
prioritizing, by the system, the plural aspects according to the varying annotation types as compared to the annotation ranking schema.

4. The computer-implemented method of claim 2, further comprising:
generating, by the system, a second identifier based on a lesser ranking second annotation type, of the varying annotation types, as compared to the annotation ranking schema,
wherein a second content of the second identifier and a content of the identifier consistently describe a same property of the chemical compound.

5. The computer-implemented method of any preceding claim, further comprising:
updating, by the system, a library datastore comprising library identifiers for chemical compounds, including the chemical compound, with the identifier for the chemical compound; and
directing, by the system, a query corresponding to the chemical compound, to the library datastore, and returns, for the same query, plural identifiers, including the identifier, having been generated for the same chemical compound based on the annotation ranking schema.

6. The computer-implemented method of claim 1, further comprising:
comparing, by the system, the identifier and a library identifier for the chemical compound, of a library datastore; and
determining, by the system, whether to update the library datastore with the identifier based on a first determination resolving a first comparison of the annotation type corresponding to the identifier and a second annotation type corresponding to the library identifier,
wherein the first comparison comprises a first sub-comparison of forms of the annotation type and the second annotation type, a second sub-comparison of rankings of the annotation type and the second annotation type as compared to the annotation ranking schema, or both.

7. The computer-implemented method of claim 6, further comprising:
determining, by the system, whether to update the library datastore with the identifier based also on a second determination resolving a second comparison of consistency of a first content of the identifier and a second content of the library identifier.

8. A computer program for facilitating a process for generating chemical compound identifiers based on varying annotation types, the computer program comprising program instructions executable by a processor to cause the processor to carry out the method steps of any preceding claim.

9. A computer program product comprising a computer readable storage medium upon which is stored the computer program of claim 8.

10. A system comprising a memory within which is stored the computer program of claim 8, and a processor that executes the program instructions of the said computer program.
